# EUROPEAN PATENT APPLICATION

(11) **EP 4 321 629 A1**
(43) Date of publication of application: **14.02.2024**
(21) Application number: 22828689.4
(22) Date of filing: 20.06.2022
(51) Int. Cl.: C12Q 1/6848

(54) **PARALLEL MULTIPLE DISPLACEMENT AMPLIFICATION METHOD**

(30) Priority: 21.06.2021 KR 20210080035
(71) Applicant: Seoul National University R&DB Foundation, Seoul 08826 (KR)
(72) Inventor: KWON, Sunghoon, Seoul 06101 (KR); LEE, Chungwon, Seoul 08796 (KR); CHOI, Ahyoun, Seoul 08785 (KR); KIM, Sungsik, Seoul 05792 (KR); KIM, Jinhyun, Seoul 08833 (KR)
(74) Representative: Prüfer & Partner mbB Patentanwälte · Rechtsanwälte
(86) International application number: PCT/KR2022/008690
(87) International publication number: WO 2022/270842

(57) **Abstract**

One aspect relates to a nucleic acid amplification method that comprises the step of amplifying nucleic acids by adding a first primer, a second primer and a nucleic acid polymerase to a sample containing a target nucleic acid. According to the nucleic acid amplification method according to one aspect, by performing parallel multiple displacement amplification using the first primer containing the barcode sequence, the increased parallelism and throughput of MDA dramatically reduces the time and cost for NGS library production, the barcode bias phenomenon can be resolved by adjusting the ratio at which the first primer is added, and the quality of amplification can also be improved using a laser-based cell separation method. Therefore, the nucleic acid amplification method can be usefully used for large-scale single-cell whole genome analysis.

## Description

### [Technical Field]

The present invention relates to a parallel multiple displacement amplification method.

### [Background Art]

Among technologies for amplifying DNA from trace amounts (amount of less than a few ng) of DNA, Multiple Displacement Amplification (MDA) is one of the widely used technologies. One of the application fields of MDA is single-cell genome analysis. Since only a very small amount of DNA exists in a single-cell, whole genome amplification technology that can amplify this to an amount that can be analyzed is important, and MDA is one of the most widely used whole genome amplification technologies. The reason why single-cell genome analysis is important is because of a phenomenon called cell heterogeneity, which means that even cells that appear identical actually have different genome base sequences. In particular, cell heterogeneity is more prominent in cancer with many mutations, brain, and immune cells. In addition, microorganisms, bacteria, and pathogens that coexist with humans or exist in the environment have much greater biological diversity than mammals, and single-cell genome analysis technology is receiving great attention in the microbiome field, which analyzes their genomes. In particular, MDA technology is important because many of the microorganisms cannot be cultured in the laboratory (unculturable), making it difficult to secure large amounts of DNA.

Another application field of MDA is the field of analyzing not only a single cell, but also multiple cells, or DNA (less than a few ng) derived from cells. In the above examples of cancer, brain, immune cells, microbiome, etc., cell heterogeneity can be identified by performing MDA amplification on multiple cells even if they are not single-cells. In addition, MDA can be useful in applications such as forensics, where samples are rare and large amounts of DNA cannot be obtained, sperms, gametes, preimplantation genetic diagnosis (PGD), preimplantation genetic screening (PGS), preimplantation genetic testing, circulating tumor cells (CTC), circulating tumor DNA, and cell free DNA. Hereinafter, for brevity of terminology, cases in which a small amount of DNA, a single cell, or a large number of cells are analyzed are collectively referred to as single-cell.

MDA has the advantage of having very high genome coverage compared to other whole genome amplification technologies, and the phi29 DNA polymerase used has high fidelity and low amplification error. Due to these two special advantages, MDA was an important technology that enabled single-cell genome analysis at the single base sequence level (single-nucleotide resolution). Here, genome analysis at the single base sequence level refers to a technology that can identify the base sequence up to the single base sequence level for more than 80% of the genome region to be analyzed. DOP-PCR, another competing technology, had high amplification uniformity, but had low genome coverage, making it difficult to apply to single-cell genome analysis at the single base sequence level. MALBAC, another competing technology, had high amplification uniformity and genome coverage, but had many single base errors (false-positive SNVs) due to the high amplification error of Bst polymerase, making it difficult to apply to genome analysis at the single base sequence level.

However, the limitation of whole genome amplification technology, such as the current MDA technology, is that as the number of cells to be analyzed increases, the cost for analysis also increases linearly. For example, there are about 10⁶ different cancer cells in 1 cm³ of cancer tissue from one patient. In order to separate all cells into individual cells and analyze them, the analysis cost is 10⁶ times higher than that of bulk sequencing, which is the existing technology, and this is a cost that is difficult to pay in reality.

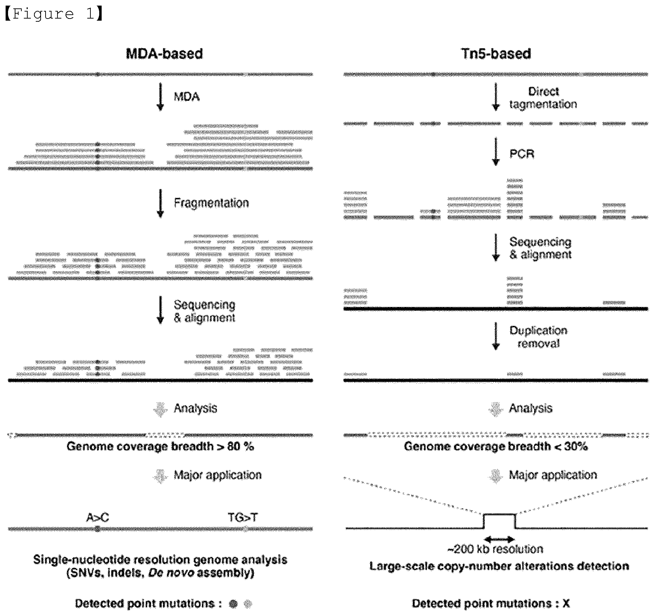

To overcome this, efforts were made to increase parallelism (multiplexity) or throughput by using another Tn5 transposase to directly tag the gDNA of a single cell and inserting a barcode or index to distinguish cells. However, the efficiency of the process was not sufficient, so sufficient genome coverage was not obtained, and single-cell genome analysis at the single base sequence level was difficult (see Fig. 1). Another competing technology has been released by Mission Bio, a product called Tapestri. Tapestri is a technology that allows detection of single nucleotide variants in hundreds of regions of a single cell by performing parallel (multiplex) PCR directly on the single cell. However, multiplex PCR-based technology has a limit to the extent of the genome area that can be analyzed simultaneously (within 100 kb), so there are limitations in analyzing wider genome areas such as the whole genome or whole exome.

Therefore, whole genome amplification technology such as MDA is essential for single-cell genome analysis at the single base sequence level. However, in order to sequence the MDA amplification product using next generation base sequence analysis technology(Next generation sequencing), each amplification product must undergo library preparation, which is a costly and time-consuming process. In other words, the more cells you want to analyze, the more you have to go through the library production process for each MDA amplification product, which leads to the problem of high library production cost and time. If a technology that can increase the parallelism and throughput of MDA amplification technology is developed, single-cell genome analysis at the single base sequence level can be performed at much lower cost and effort, making it possible to analyze a much larger number of heterogeneous cells.

The problem that library production costs increase as a large number of single-cells are analyzed is a common difficulty experienced in all single-cell analysis fields. Similar difficulties have been encountered in all fields, including single-cell transcriptome (transcriptomics), single-cell epigenome (epigenetics), single-cell proteome (proteomics), and single-cell multiome (multiomics), and the most widely used and effective means to overcome this problem is parallelization technology using barcoding. The library production process has two main purposes as follows: 1) The process of inserting an adapter so that the DNA to be analyzed can be sequenced in a next generation base sequence analyzer, and 2) The process of inserting a barcode or index to identify samples when multiple samples are sequenced simultaneously. In the field of single-cell analysis, different barcodes must be inserted into different cells to distinguish them. In other words, to achieve the purpose of 2), each individual cell must undergo a library preparation process, respectively, which increases the cost of the library preparation process. However, if the purpose of 2) can be achieved before the library production process, the purpose of 1) can be achieved by going through the library production process for DNA derived from multiple cells at once. In other words, the above-mentioned parallelization technology using barcoding performs barcoding in advance on biological molecules such as DNA, RNA, protein, and epigenome derived from a single cell. Therefore, it is a technology that can innovatively reduce cost, time, and labor by pooling all biological molecules derived from different cells and performing the one-pot library production process.

In fact, in many single-cell analysis fields such as single-cell transcriptome (transcriptomics), single-cell epigenome (epigenetics), single-cell proteome (proteomics) and single-cell multiome (multiomics), analysis parallelism and throughput have been innovatively increased by using the above-mentioned barcoding parallelization technology. Even when the goal was to examine the single-cell genome sparsely, hundreds to tens of thousands of cells could be analyzed in parallel using the method using the Tn5 transposase mentioned above. Here, examining the genome sparsely refers to sparsely examining a portion of the genome lower than 80%, rather than examining the entire genome of a single-cell.

However, for single-cell genome analysis at the single base sequence level, barcodes must be inserted into only a few copies of DNA (for humans, 2 copies) without loss, which is a difficult goal to achieve. For parallelization technology using barcoding to be effective, the base sequence of the barcode and the base sequence derived from the cell genome to be analyzed must be read simultaneously in NGS sequencing to match the information of the sequenced base sequence derived from the cell with the barcode information. However, Illumina's NGS technology, which is currently the most widely used, has a maximum nucleic acid read length limit of about 300 to 600 bp that can be sequenced at one time, and inserting a barcode so that the barcode exists without loss even when the DNA of a single cell is fragmented into such short nucleic acid fragments is a difficult task to achieve. Unlike DNA, RNA usually has an A tail at the end of the transcript, making it easy to insert a barcode for the entire transcript by inserting it with a PCR (polymerase chain reaction) handle. However, because DNA is composed of long strands of very complex base sequences, there is no specific sequence that can be used as a PCR handle, and it is difficult to insert barcodes into the entire genome without loss.

In other words, in order to analyze a single-cell genome of which there are only a few copies at the single nucleotide level and at the same time apply parallelization technology using barcode, a process such as MDA, which pre-amplifies a small amount of single-cell genome, is essential to achieve lossless barcoding of the entire genome region. However, introducing parallelization technology using barcoding to MDA technology is also difficult to achieve. In a typical PCR reaction, adding a barcode to the 5' end of the primer to introduce parallelization technology using barcoding did not significantly affect the PCR reaction, but MDA, a technology that usually uses N6 (random hexamer) as a primer, has a significant negative impact on MDA reaction efficiency if another sequence is added to the end of the 5' primer. The cause of this phenomenon is not yet known to the academic world, and the problem cannot be solved with simple solutions such as increasing the cation concentration or inserting a hairpin structure to reduce non-specific hybridization of the primer. Therefore, it was difficult to insert a barcode during the MDA process, so the subsequent library preparation process had to be performed for each individual cell.

Recently, a whole genome amplification technology called LIANTI, which is different from MDA, has been developed, and it has been claimed that a barcode can be directly inserted into the DNA fragment resulting from LIANTI. However, LIANTI's DNA fragments range in length from 100 bp to 2 kb. Therefore, there is a limitation that it is difficult to read all of the barcoded LIANTI library without fragmentation with sequencing of 300 to 600 bp, which is the read length limit of current short-read sequencing technology. However, because the barcode information of the DNA molecule is lost when fragmentation is performed, LIANTI technology is difficult to parallelize when the purpose is to analyze the single-cell genome at the single base sequence level.

Meanwhile, another difficulty with MDA technology is that when amplification is performed from a small amount of DNA, such as in a single-cell, amplification bias is severe, making it difficult to obtain high-quality amplification results. Typically, when 100 single-cells are amplified by MDA, only about 5 to 10 of them show results that can be analyzed for gene copy number alteration (CNA), and all remaining cells do not pass QC (Quality Control) and are discarded. This problem is called MDA's amplification bias problem, or amplification quality problem. As a result, in order to analyze a certain number of cells, MDA amplification had to be performed on 10 to 20 times more cells, which resulted in cost inefficiency and made it difficult to analyze a large amount of single-cells using MDA.

Therefore, by increasing the parallelism and throughput of MDA, a parallel multiple displacement amplification method using barcode-containing primers was developed so that it can be usefully used for large-scale single-cell whole genome analysis.

### [Prior Art Document]

### [Patent Document]

Korean Laid-open Patent Publication No. 10-2015-0016036

### [Disclosure]

### [Technical Problem]

One aspect is to provide a nucleic acid amplification method comprising the step of amplifying nucleic acids by adding a first primer, a second primer and a nucleic acid polymerase to a sample containing a target nucleic acid, wherein
the first primer is a polynucleotide consisting of a base sequence including a first base sequence that hybridizes to the target nucleic acid; and
a fixed second base sequence that distinguishes the sample from which the target nucleic acid is derived, and
the second primer is a polynucleotide consisting of a base sequence including a third base sequence that hybridizes to the target nucleic acid.

Another aspect is to provide a primer set for nucleic acid amplification comprising a first primer and a second primer, wherein
the first primer is a polynucleotide consisting of a base sequence including a first base sequence that hybridizes to the target nucleic acid; and
a fixed second base sequence that distinguishes the sample from which the target nucleic acid is derived, and
the second primer is a polynucleotide consisting of a base sequence including a third base sequence that hybridizes to the target nucleic acid.

Another aspect is to provide a kit for nucleic acid amplification comprising a first primer and a second primer, wherein
the first primer is a polynucleotide consisting of a base sequence including a first base sequence that hybridizes to the target nucleic acid; and
a fixed second base sequence that distinguishes the sample from which the target nucleic acid is derived, and
the second primer is a polynucleotide consisting of a base sequence including a third base sequence that hybridizes to the target nucleic acid.

### [Technical Solution]

One aspect provides a nucleic acid amplification method comprising the step of amplifying nucleic acids by adding a first primer, a second primer and a nucleic acid polymerase to a sample containing a target nucleic acid, wherein
the first primer is a polynucleotide consisting of a base sequence including a first base sequence that hybridizes to the target nucleic acid; and
a fixed second base sequence that distinguishes the sample from which the target nucleic acid is derived, and
the second primer is a polynucleotide consisting of a base sequence including a third base sequence that hybridizes to the target nucleic acid.

The "target nucleic acid" refers to a nucleic acid that can be amplified by the nucleic acid amplification method and sequenced through the NGS (Next generation sequencing) library preparation process, and may include the whole genome. According to the nucleic acid amplification method according to one aspect, the target nucleic acid can be sequenced at the single base sequence level.

In addition, according to the nucleic acid amplification method according to one aspect, by performing parallel multiple displacement amplification using the first primer and the second primer, NGS library preparation time and cost can be innovatively reduced by increasing the parallelism and throughput of multiple displacement amplification.

The target nucleic acid may be selected from DNA, RNA, genomic DNA, cDNA, exon, intron, part of a gene, part of plasmid, regulatory sequence, circular RNA, cell-free DNA (cfDNA), cell-free RNA (cfRNA), small interfering RNA (siRNA), cell-free fetal DNA (cffDNA), mRNA, tRNA, rRNA, miRNA, snoRNA, piRNA, ncRNA, locked nucleic acid (LNA), peptide nucleic acid (PNA), synthetic polynucleotide, polynucleotide analog, or a combination thereof.

The target nucleic acid may be single-stranded, double-stranded, or triple-stranded. Additionally, only a portion of the target nucleic acid may be double-stranded or triple-stranded.

The target nucleic acid may be composed of a completely unknown base sequence, a partially known base sequence, or a completely known base sequence.

The target nucleic acid may be composed of a polynucleotide consisting of naturally occurring bases (uracil, adenine, thymine, cytosine, guanine), or a polynucleotide analog.

The polynucleotide analog may include i) a modified base, ii) a modified pentose, iii) a modified phosphate, or iv) a linking portion between modified bases.

The modified base may include diaminopurine, isocytosine, isoguanine, pseudoisocytosine, N9-(2-amino-6-chloropurine), N9-(2,6-diaminopurine), N9-(7-deaza-guanine), N9-(7-deaza-8-azaguanine) and N8-(7-deaza-8-aza-adenine), 5-propynyl-uracil, 2-thio-5-propynyl-uracil, 5-fluorouracil, 5-bromouracil, 5-chlorouracil, 5-iodouracil, hypoxanthine, xanthine, 4-acetylcytosine, 5-(carboxyhydroxylmethyl)uracil, 5-carboxymethylaminomethyl-2-thiouridine, 5-carboxymethylaminomethyluracil, dihydrouracil, beta-D-galactosylqueosin, inosine, N6-isopentenyladenine, 1-methylguanine, 1-methylinosine, 2,2-dimethylguanine, 2-methyladenine, 2-methylguanine, 2-thiothymine, 2-aminopurine, 3- Methyl cytosine, 5-methylcytosine, N6-adenine, 7-methyl guanine, 5-methylaminomethyluracil, 5-methoxyaminomethyl-2-thiouracil, beta-D-mannosylqueosin, 5'-methoxycarboxymethyluracil, 5-methoxyuracil, 2-methylthio-D46-isopentenyladenine, uracil-5-oxyacetic acid (v), wybutoxosine, pseudouracil, queosin, 2-thiocytosine, 5-methyl-2-thiouracil, 2-thiouracil, 4-thiouracil, 5-methyluracil, uracil-5-oxyacetic acid methyl ester, uracil-5-oxyacetic acid (v), 5-methyl-2-thiouracil, 3-(3-amino-3-N-2-carboxypropyl) uracil, (acp3)w, oligonucleotide phosphoramidate, nitropyrrole, nitroindole , acylic 5-nitroindole, 4-nitropyrazole, 4-nitroimidazole, C-5 propynyl-C, C-5 propynyl-U or 2-amino adenine.

The modified pentose may be a locked nucleic acid (LNA) analog (e.g., Bz-A-LNA, 5-Me-Bz-C-LNA, dmf-G-LNA or T-LNA, etc.) or a 2'- or 3'-variant, where the 2'- or 3'-position is hydrogen, hydroxy, alkoxy (e.g., methoxy, ethoxy, allyloxy, isopropoxy, butoxy, isobutoxy and phenoxy), azido, amido, alkylamino, fluoro, chloro or bromo).

The modified phosphate may be a phosphate analog in which the phosphorus atom is in the +5 oxidation state and one or more of the oxygen atoms are replaced with a non-oxygen moiety, such as sulfur. Still other modified phosphates may include phosphorothioate, phosphorodithioate, phosphoroselenoate, phosphorodiselenoate, phosphoroanilothioate, phosphoanilidate, phosphoramidate or boronophosphate, including associated counterions such as H⁺, NH₄⁺, Na⁺.

The linking portion between the modified bases may be a phosphate analog, an achiral analog, an uncharged morpholino-based polymer with uncharged intersubunit linkages or achiral intersubunit linkages. Some base-to-base linkage analogs may be morpholidate, or acetal- or polyamide-linked heterocycles. Additionally, when the base is a peptide nucleic acid (PNA), the linking portion between the modified bases may be a 2-aminoethylglycine amide skeletal polymer.

The target nucleic acid may be derived from a single-cell or multiple cells, may be biochemically treated (e.g., bisulfite or reverse transcription), or may remain in the environment.

The sample may include the target nucleic acid, and the sample may be one or more samples selected from the group consisting of cell samples, tissue samples, blood samples, urine samples, saliva samples, lymph fluid samples, cerebrospinal fluid samples, amniotic fluid samples, pleural fluid samples, pericardial fluid samples, ascites samples, aqueous humor samples, bone marrow samples, semen samples, biopsy samples, cancer samples, tumor samples, forensic samples, archaeological samples, paleontological samples, infection samples, production samples, plant samples, microorganism samples, virus samples, soil samples, marine samples and freshwater samples.

The tissue sample may be derived from one or more tissues selected from the group consisting of epididymis, eyes, muscles, skin, tendons, veins, arteries, blood, heart, spleen, lymph nodes, bone, bone marrow, lungs, bronchi, trachea, intestines, small intestine, large intestine, colon, rectum, salivary glands, tongue, bladder, appendix, liver, pancreas, brain, stomach, skin, kidneys, ureters, bladder, urethra, sex glands, testes, ovaries, uterus, fallopian tubes, thymus, pituitary gland, thyroid gland, adrenal glands and parathyroid glands. Additionally, the tissue sample may be derived from any of a variety of organs of a human or other organism.

The cell sample may be derived from animal cells, plant cells, fungal cells, bacterial cells or protozoan cells, and may specifically be derived from animal cells, and more specifically, may be derived from human cells.

The cell sample may be derived from one or more cells selected from the group consisting of germ cells (egg cells, sperm, etc.), ovarian epithelial cells, ovarian fibroblasts, immune cells, B cells, T cells, natural killer cells, dendritic cells, cancer cells, eukaryotic cells, stem cells, blood cells, muscle cells, fat cells, skin cells, nerve cells, bone cells , pancreatic cells, endothelial cells, pancreatic epithelial cells, pancreatic alpha cells, pancreatic beta cells, pancreatic endothelial cells, bone marrow lymphoblasts, bone marrow B lymphoblasts, bone marrow macrophages, bone marrow erythroblasts, bone marrow dendritic cells, bone marrow adipocytes, bone marrow osteocytes, bone marrow chondrocytes, preosteoblasts, bone marrow megakaryoblasts, brain B lymphocytes, brain glial cells, neurons, brain astrocytes, neuroectodermal cells, brain macrophages, brain microglia, brain epithelial cells, cortical neurons, brain fibroblasts, mammary epithelial cells, colon epithelial cells, colonic B lymphocytes, breast myoepithelial cells, breast fibroblasts, colonic enterocytes, cervical epithelial cells, ductal epithelial cells, tongue epithelial cells, tonsil dendritic cells, tonsil B lymphocytes, peripheral blood lymphoblasts, peripheral blood T lymphoblasts, peripheral blood skin T lymphocytes, peripheral blood natural killer cells, peripheral blood B lymphoblasts, peripheral blood monocytes, peripheral blood myeloblasts, peripheral blood mononuclear blasts, peripheral blood preosteoblasts, peripheral blood macrophages, peripheral blood basophils, liver endothelial cells, liver mast cells, liver epithelial cells, liver B lymphocytes, spleen endothelial cells, spleen epithelial cells, spleen B lymphocytes, liver cells, liver fibroblasts, lung epithelial cells, bronchial epithelial cells, lung fibroblasts, lung B lymphocytes, lung Schwann cells, lung squamous cells, lung macrophages, lung osteoblasts, neuroendocrine cells, alveoli, gastric epithelial cells, gastric fibroblasts, stem cells, fetal cells, tumor cells, suspicious cancer cells, cancer cells and cells that have undergone a gene editing procedure.

In one aspect, the sample may be separated by at least one technique selected from the group consisting of spatially resolved technique, dielectrophoretic digital sorting, fluorescence activated cell sorting(FACS), hydrodynamic traps, droplet, microwell, microfluidics, micromanipulation, Raman tweezers and CellRaft. The above technologies can classify samples according to specific criteria (e.g., location in space, presence or absence of surface proteins, H&E staining images or in-situ sequencing images, etc.), and can analyze the genome of each sample at the single base sequence level by performing the nucleic acid amplification method on each classified sample. Additionally, the technology can increase the amount of input gDNA, thereby improving the amplification quality of the nucleic acid amplification method.

According to one aspect, the liquid droplet, microwell or microfluidics technology can dramatically increase the number of physically compartmentalized reactors for biochemical reactions, thereby dramatically increasing the parallelism of the nucleic acid amplification method. Specifically, by trapping a single-cell inside the liquid droplet or microwell and then performing the nucleic acid amplification method in each compartmented solution, a large amount of single-cells can be analyzed at once.

Further, in one aspect, the spatially resolved technique may be at least one technique selected from the group consisting of Laser capture microdissection (LCM), laser microdisection (LMD), Laser pressure catapulting (LPC) and phenotype-based high-throughput laser-aided isolation and sequencing (PHLI-seq).

In one aspect, the cell sample may be lysed using proteinase, protease, heat, bead beating, sanitation, alkaline dissolution above 50°C or alkaline dissolution at temperatures below 4°C. Additionally, the target nucleic acid may be denatured into a single strand using heat or alkaline denaturation for amplification.

The term "amplification" refers to any action in which all or part of a nucleic acid is copied into an additional nucleic acid, and can be used in the same sense as "replication." The additional nucleic acid may include a sequence that is substantially identical to or substantially complementary to a portion of the template nucleic acid. The additional nucleic acid or template nucleic acid may each independently be single stranded or double stranded. Additionally, the amplification may include linear amplification or exponential amplification of nucleic acids.

The "nucleic acid amplification method" may be multiple displacement amplification (MDA), and specifically may be parallel multiple displacement amplification. The parallel multiple displacement amplification can be used interchangeably with "barcode MDA (bMDA)".

The term "multiple displacement amplification" refers to a method of exponentially amplifying nucleic acids using nucleic acid polymerase with a strand displacement function.

In one aspect, through the nucleic acid amplification method, nucleic acid polymerase can replicate the target nucleic acid to obtain a replicated nucleic acid strand, and the replicated nucleic acid strand can be displaced from the target nucleic acid.

According to one aspect, the nucleic acid amplification method can analyze a single-cell genome at the single base sequence level. In addition, the above method can be used in single-cell multiome (multiomics) analysis technology by performing it together with technology to analyze single-cell transcriptome, epitranscriptome, proteome, etc. The single-cell multiome analysis technology can be used to simultaneously analyze various information about individual cells by analyzing two-dimensional or more omics information. Therefore, the nucleic acid amplification method according to one aspect can analyze a single-cell at the single base sequence level with high throughput, thereby improving the throughput of single-cell multiome analysis technology.

The "first primer" is a polynucleotide containing the first base sequence that hybridizes to the target nucleic acid and the fixed second base sequence that distinguishes the sample from which the target nucleic acid is derived.

The "hybridization" may refer to the process of forming a stable double-stranded polynucleotide by non-covalently combining two single strand polynucleotides.

In one aspect, the number of bases in the first base sequence may be an integer selected from 4 to 10. The first base sequence may be designed to hybridize with the target nucleic acid.

Additionally, in one aspect, the bases constituting the first base sequence may be bases selected from the group consisting of A (adenine), T (thymine), G (guanine), C (cytosine), and modifications thereof.

The modification may be one or more selected from the group consisting of diaminopurine, isocytosine, isoguanine, pseudoisocytosine, N9-(2-amino-6-chloropurine), N9-(2,6-diaminopurine), N9-(7-deaza-guanine), N9-(7-deaza-8-aza-guanine) and N8-(7-deaza-8-aza-adenine), 5-propynyl-uracil, 2-thio-5-propynyl-uracil, 5-fluorouracil, 5-bromouracil, 5-chlorouracil, 5-iodouracil, hypoxanthine, xanthine, 4-acetylcytosine, 5-(carboxyhydroxylmethyl)uracil, 5-carboxymethylaminomethyl-2-thiouridine, 5-carboxymethylaminomethyluracil, dihydrouracil, beta-D-galactosylqueosin, inosine, N6-isopentenyladenine, 1-methylguanine, 1-methylinosine, 2,2-dimethylguanine, 2-methyladenine, 2-methylguanine, 2-thiothymine, 2-aminopurine, 3- Methyl cytosine, 5-methylcytosine, N6-adenine, 7-methyl guanine, 5-methylaminomethyluracil, 5-methoxyaminomethyl-2-thiouracil, beta-D-mannosylqueosin, 5'-methoxycarboxymethyluracil, 5-methoxyuracil, 2-methylthio-D46-isopentenyladenine, uracil-5-oxyacetic acid (v), wybutoxosine, pseudouracil, queosin, 2-thiocytosine, 5-methyl-2-thiouracil, 2-thiouracil, 4-thiouracil, 5-methyluracil, uracil-5-oxyacetic acid methyl ester, uracil-5-oxyacetic acid (v), 5-methyl-2-thiouracil, 3-(3-amino-3-N-2-carboxypropyl) uracil, (acp3)w, oligonucleotide phosphoramidate, nitropyrrole, nitroindole , acylic 5-nitroindole, 4-nitropyrazole, 4-nitroimidazole, C-5 propynyl-C, C-5 propynyl-U or 2-amino adenine.

According to one aspect, the first base sequence may be fixed to a specific base sequence depending on the target nucleic acid, may be composed of various base sequences, or may be composed of a random base sequence. In addition, the first base sequence may be a random base sequence with a specific fixed base sequence added to facilitate binding with the target nucleic acid.

Additionally, in one aspect, the first base sequence may include a base that is resistant to nuclease. The base may be phosphorothiolate modified. Through the above modification, when amplifying the target nucleic acid with nucleic acid polymerase that has the function of decomposing 3'-5' nucleic acids, primer depletion due to nucleic acid decomposition can be prevented.

In one aspect, the number of bases in the second base sequence may be an integer selected from 4 to 35. Additionally, the number of types of the second base sequence may be 10 to 1,000,000. The second base sequence can distinguish target nucleic acids from a large amount of different samples at once.

Additionally, in one aspect, the second base sequence may include a barcode sequence. The number of bases in the barcode sequence may be an integer selected from 4 to 12. The barcode sequence is designed to have the same base sequence for the target nucleic acid of a specific sample and a different base sequence for the target nucleic acid of other samples, so that the sample from which the target nucleic acid is derived can be distinguished.

In one aspect, "the first primer" may include two or more of the second base sequence.

In one aspect, "the second primer" is a polynucleotide containing the third base sequence that hybridizes to the target nucleic acid.

In one aspect, the number of bases in the third base sequence may be an integer selected from 4 to 10. The third base sequence may be designed to hybridize with the target nucleic acid.

Additionally, in one aspect, the bases constituting the third base sequence may be bases selected from the group consisting of A (adenine), T (thymine), G (guanine), C (cytosine), and modifications thereof.

According to one aspect, the third base sequence may be fixed to a specific base sequence depending on the target nucleic acid, may be composed of various base sequences, or may be composed of a random base sequence. In addition, the third base sequence may be a random base sequence with a specific fixed base sequence added to facilitate binding with the target nucleic acid.

Additionally, in one aspect, the third base sequence may include a base that is resistant to nuclease. The base may be phosphorothiolate modified. Through the above modification, when amplifying the target nucleic acid with nucleic acid polymerase that has the function of decomposing 3'-5' nucleic acids, primer depletion due to nucleic acid decomposition can be prevented.

In one aspect, the first primer may be a combined with a substance that inhibits the affinity between the nucleic acid polymerase and the first primer. Specifically, the substance may be a physical structure that exhibits steric hindrance. The substance can increase nucleic acid amplification efficiency by preventing binding by inhibiting the affinity between the nucleic acid polymerase and the first primer through steric hindrance.

In addition, in one aspect, by manufacturing the barcode sequence region with RNA, adding a linker between the barcode sequence region and the binding region, or hybridizing single-strand DNA (ssDNA) of a sequence complementary to the barcode region, the affinity between the first primer and the nucleic acid polymerase can be inhibited, thereby increasing nucleic acid amplification efficiency.

In one aspect, the nucleic acid amplification method may further include the step of selecting the nucleic acid amplified with the first primer.

In one aspect, the first primer may be combined with a capture material. After amplifying the nucleic acid with the first primer combined with the capture material and then capturing the capture material in the amplified nucleic acid, a target nucleic acid containing the second base sequence and a target nucleic acid not containing the second base sequence can be selected.

In one aspect, the first primer can be synthesized individually to include all of the first base sequence, the second base sequence, and the capture material; after synthesizing only the first base sequence and the second base sequence, additional capture material may be added through nucleic acid modification; and the first base sequence and the second base sequence can be synthesized and connected through ligation or chemical reaction, and then additional capture materials can be added through nucleic acid modification.

In one aspect, the capture material may be one or more selected from the group consisting of biotin, D-biotin, biotin dT, biotin-TEG, dual biotin, PC-biotin, acrylic, thiol, dithiol, amine group, acrylic group, NHS ester, azide, hexynyl, octadiynyl dU, his tag, protein tag, poly adenine (polyA), at least 10 base sequences among the base sequences that make up the NGS sequencing adapter, and a polynucleotide consisting of at least 10 base sequences. Specifically, it may be one or more selected from the group consisting of biotin, D-biotin, biotin dT, biotin-TEG, dual biotin, and PC-biotin, and more specifically, it may be D-biotin.

In addition, in one aspect, the bases constituting at least 10 base sequences may be bases selected from the group consisting of A (adenine), T (thymine), G (guanine), C (cytosine), and modifications thereof.

According to one aspect, when a molecule other than nucleic acid is used as the capture material, the inhibition of the strand displacement reaction by long-length primers can be greatly reduced, and the strand displacement replication efficiency can be significantly increased.

In addition, in one aspect, when biotin is used as the capture material, PCR bias phenomenon and amplification errors can be minimized because PCR is not used to selectively enrich the target nucleic acid containing the barcode sequence. The PCR bias phenomenon refers to a phenomenon in which a specific nucleic acid is amplified more or less than other nucleic acids during the PCR process, and amplification error refers to a phenomenon in which the nucleic acid base sequence is copied differently from the original due to an error in nucleic acid polymerase during the PCR process.

In one aspect, the NGS sequencing adapter may be a partial base sequence of a primer for NGS sequencing. By adding the adapter to the first primer and performing PCR, only amplification products containing the barcode sequence can be concentrated. In addition, 10 or more base sequences of the base sequences constituting the NGS sequencing adapter, that is, some of the NGS sequencing adapters, may be used as the capture material.

In one aspect, other fixed base sequences may be further added to the first primer. For example, in the case of Multiple Annealing and Looping Based Amplification Cycles (MALBAC) amplification technology, in addition to the random base sequence consisting of 8 bases to amplify the target nucleic acid, a specific fixed base sequence that can form a loop is added. MALBAC can perform a DNA amplification reaction including strand displacement using the primer, and then performs PCR using a primer having a specific fixed base sequence. During this PCR process, the amplification product having a specific fixed base sequence at both ends forms a loop, thereby reducing amplification bias. In addition, the nucleic acid amplification method according to one aspect also allows amplification of the target nucleic acid using the first primer to which a fixed base sequence for a specific purpose is added. As a simple example, the target nucleic acid can be amplified using the first primer, which further contains a barcode base sequence between the fixed base sequence of the MALBAC primer and the random base sequence. In the above example, since a specific fixed base sequence can take the role of capture material, the first primer without additional capture material may be used. The number of bases in the specific fixed base sequence may vary depending on the purpose, but may generally be an integer selected from 5 to 30.

In one aspect, the first primer may be modified to tag one or more fluorescent materials selected from the group consisting of fluorescein isothiocyanate, 5,6-carboxymethyl fluorescein, Texas red, 2-NBDG (nitrobenz-2-oxa-1,3) -diazol-4-yl), coumarin, dansyl chloride, rhodamine, amino-methyl coumarin, Eosin, Erythrosin, BODIPY, Cascade Blue, Oregon Green, pyrene, lissamine, xanthene, acridine, oxazines, phycoerythrin, Cy3, Cy3.5, Cy5, Cy5.5, and Cy7. Through this, the amount of barcoded amplification product among nucleic acid amplification reaction products can be quantified.

In one aspect, the first primer may include a UMI(Unique Molecular Identifier). The UMI refers to a sequence containing a wide variety of nucleic acids with unique base sequences for inserting a molecular barcode with a unique base sequence for each target nucleic acid molecule during the nucleic acid amplification process. By inserting UMI during the amplification process, it is possible to determine whether the base sequence obtained from the amplification product is a duplicate sequence resulting from an amplification bias or a sequence derived from a different target molecule. Through this, the first primer containing UMI can reduce amplification bias.

According to one aspect, the selection may be performed by a method using one or more substances selected from the group consisting of avidin, neutravidin, streptavidin, carboxylic group, thiol, alkyne, digoxigenin, azide and hexynyl, or a Polymerase Chain reaction (PCR) method. Specifically, it may be performed by a method using one or more substances selected from the group consisting of avidin, neutravidin and streptavidin, and more specifically, it may be performed by a method using streptavidin.

According to one aspect, the streptavidin can bind strongly to biotin and can select target nucleic acids to which biotin is bound. In addition, the streptavidin can reversibly bind to biotin, so the amplified target nucleic acid containing the barcode sequence can be recovered after selection.

Additionally, in one aspect, when D-biotin is used as the capture material, the amplified target nucleic acid can be effectively obtained with streptavidin without denaturing.

In one aspect, the nucleic acid amplification method may further comprise the steps of: purification of the nucleic acid amplified from the sample;
fragmentation of the purified nucleic acid; and
preparation of Next Generation Sequencing library using the fragmented nucleic acid.

The "purification" may be performed using solid phase reversible immobilization (SPRI) technique. Specifically, 0.8x sample volume of Ampure magnetic beads can be added, and the nucleic acid can be purified according to the protocol.

The "fragmentation" can be performed using sonication or enzymes. In addition, the nucleic acid may be fragmented to a length of 50 bp to 10 kb.

The "purification step" may be performed before or after the selection step. When the "purification step" is performed before the selection step, the "fragmentation step" may be performed before or after the selection step.

In one aspect, the selection may be selecting double-stranded or single-stranded nucleic acids. If the selected nucleic acid is double-stranded, the NGS library can be prepared by removing biotin from the double-stranded nucleic acid, or the NGS library can be prepared with biotin bound. When the NGS library is manufactured with biotin bound to the 5'-end, the NGS adapter cannot be ligated to the nucleic acid strand where 5'-biotin is present, and the adapter is ligated only to the opposite strand. Therefore, the position where the barcode sequence appears in the NGS sequencing results can be fixed.

In one aspect, the first primer may be added at 0.1 to 10 mol% compared to the second primer. Specifically, the first primer may be added at 0.1 to 1 mol%, 0.1 to 3 mol%, 0.1 to 5 mol%, 0.1 to 7 mol%, 0.1 to 10 mol%, 1 to 3 mol%, 1 to 5 mol%, 1 to 7 mol%, 1 to 10 mol%, 3 to 5 mol%, 3 to 7 mol%, 3 to 10 mol%, 5 to 7 mol%, 5 to 10 mol% or 7 to 10 mol% compared to the second primer.

According to one aspect, the efficiency of the nucleic acid amplification method can be increased by adjusting the addition ratio of the first primer and the second primer.

In addition, according to one aspect, the amplification bias of the nucleic acid amplification method can be reduced by adjusting the addition ratio of the first primer and the second primer. Specifically, by adjusting the addition ratio of the first primer and the second primer, the target nucleic acid for each sample can be recovered in a constant amount in the selection step, and since the sequencing reads for each sample can be consistently adjusted during the NGS library manufacturing stage, the amplification bias of the nucleic acid amplification method can be reduced. Additionally, the ratio can be adjusted differently depending on the type of the second base sequence.

In one aspect, the nucleic acid polymerase may be DNA polymerase or RNA polymerase. Additionally, the DNA polymerase may be one or more DNA polymerases selected from the group consisting of phi29 DNA polymerase, Tts DNA polymerase, phase M2 DNA polymerase, VENT DNA polymerase, Klenow fragment of DNA polymerase I, T5 DNA polymerase, PRD1 DNA polymerase, T4 DNA polymerase holoenzyme, T7 polymerase-derived T7 Sequenase and Bst DNA polymerase. Specifically, it may be one or more DNA polymerases selected from the group consisting of phi29 DNA polymerase and Bst DNA polymerase, and more specifically, it may be phi29 DNA polymerase.

In one aspect, the DNA polymerase may be added in an amount capable of polymerizing 0.1 to 10 nmol or more dNTPs per 10 seconds to 10 minutes, and specifically, may be added in an amount capable of polymerizing 1 nmol or more dNTPs per minute.

In one aspect, in the nucleic acid amplification method, the samples may be plural and different from each other,
the target nucleic acids may be plural and different from each other,
the first primers may be plural and different from each other,
the second primers may be plural and different from each other, and
the first primers, which are plural and different from each other, may be polynucleotides composed of base sequences including the second base sequences, which are fixed and different from each other.

According to one aspect, by performing the nucleic acid amplification method multiple times with the plurality of the first primers containing the second base sequences, which are fixed and different from each other, the target nucleic acid derived from different samples can be distinguished.

In one aspect, the first primer may be added at 0.1 to 10 mol% compared to the second primer. Specifically, the first primer may be added at 0.1 to 1 mol%, 0.1 to 3 mol%, 0.1 to 5 mol%, 0.1 to 7 mol%, 0.1 to 10 mol%, 1 to 3 mol%, 1 to 5 mol%, 1 to 7 mol%, 1 to 10 mol%, 3 to 5 mol%, 3 to 7 mol%, 3 to 10 mol%, 5 to 7 mol%, 5 to 10 mol% or 7 to 10 mol% compared to the second primer.

According to one aspect, by adjusting the addition ratio of the first primer and the second primer, the efficiency of the nucleic acid amplification method can be increased.

In addition, according to one aspect, the amplification bias of the nucleic acid amplification method can be reduced by adjusting the addition ratio of the first primer and the second primer. Specifically, by adjusting the addition ratio of the first primer and the second primer, the target nucleic acid for each sample can be recovered in a constant amount in the selection step, and since the sequencing reads for each sample can be consistently adjusted during the NGS library manufacturing stage, the amplification bias of the nucleic acid amplification method can be reduced. Additionally, the ratio can be adjusted differently depending on the type of the second base sequence.

In one aspect, the nucleic acid amplification method may further include the step of selecting nucleic acids amplified with the plurality of the first primers.

Additionally, in one aspect, the plurality of the first primers may be combined with capture material. The "capture material" may be within the range described above.

In one aspect, the nucleic acid amplification method may further comprise the steps of: purification of the nucleic acids amplified from the plurality of the samples;
fragmentation of the purified nucleic acids;
pooling of the fragmented nucleic acids; and
preparation of Next Generation Sequencing library using the pooled nucleic acids.

The "purification step" and "fragmentation step" may be performed before or after the selection step. When the "purification step" is performed before the selection step, the "fragmentation step" may be performed before or after the selection step.

In one aspect, the "pooling step" refers to a step of collecting different target nucleic acid amplicons obtained from a plurality of the first primers containing different fixed second base sequences into one pool at a time. The pooling step can be performed at any point desired by the user before or after the selection step, before or after the purification step, before or after the fragmentation step, or before the NGS library preparation step. Additionally, in the pooling step, if the user wants to analyze a larger amount of sequencing reads, the amount of pooling for each sample can be adjusted by pooling more samples and pooling less samples if the user wants to analyze a smaller amount.

In one aspect, the nucleic acid amplification method may be performed by further comprising a nucleic acid purification step after the pooling step.

In addition, in one aspect, in order to increase the throughput of the nucleic acid amplification, the first primer with the same second base sequence was added to each liquid droplet or microwell and the nucleic acid amplification method was performed. By doing so, more than a thousand types of samples can be amplified at once and then pooled at once (one-pot).

According to one embodiment, it was confirmed that by performing a nucleic acid amplification method consisting of a cell lysis step, a bMDA step, a bMDA amplification product processing step, and a bMDA-seq step, an NGS library can be prepared for DNA derived from multiple single-cells at once. The method was performed by adding a primer containing a barcode sequence (the first primer) in addition to the random base sequence primer (the second primer) commonly used in MDA reactions, and based on the barcode sequence, it is possible to determine which sample the corresponding NGS read originated from. Therefore, the parallelism and throughput of the MDA reaction can be significantly increased (see Example 1).

According to another embodiment, in the nucleic acid amplification method, it was confirmed that the barcode bias phenomenon can be reduced by adjusting the addition ratio of the primer containing the barcode sequence (the first primer). Specifically, the barcode bias phenomenon may mean bMDA amplification bias, bias in the amount of barcoding DNA contained in the bMDA amplification product, or bias in NGS library conversion efficiency. Specifically, it was confirmed that if the ratio of all barcode-containing primers is constant, the CV value is about 29.9%, whereas if the ratio of barcode-containing primers is set differently to a specific ratio, the CV value can be reduced up to about 7.68%. Therefore, it was confirmed that when the addition ratio of the primer containing the barcode sequence was set differently to a specific ratio, the barcode bias phenomenon was significantly reduced (see Example 2).

According to still another embodiment, it was confirmed that the amplification quality of bMDA improved when a laser-based cell separation method was used as a method of separating cell samples. The laser-based cell separation method can minimize the amplification bias phenomenon of bMDA and provide high-quality amplification products by separating and analyzing physically adjacent surrounding cells (see Example 3).

Another aspect provides a primer set for nucleic acid amplification comprising the first primer and the second primer, wherein
the first primer is a polynucleotide consisting of a base sequence including a first base sequence that hybridizes to the target nucleic acid; and
a fixed second base sequence that distinguishes the sample from which the target nucleic acid is derived, and
the second primer is a polynucleotide consisting of a base sequence including a third base sequence that hybridizes to the target nucleic acid.

The terms "first primer", "second primer", "target nucleic acid", "sample", "first base sequence", "second base sequence" and "third base sequence" may be within the aforementioned range.

According to one aspect, when performing the nucleic acid amplification method with the above primer set, target nucleic acids derived from different samples can be distinguished.

In one aspect, in the primer set,
the first primers may be plural and different from each other,
the second primers may be plural and different from each other, and
the first primers, which are plural and different from each other, may be polynucleotides composed of base sequences including the second base sequences, which are fixed and different from each other.

According to one aspect, the first primer in the above primer set may be added at 0.1 to 10 mol% compared to the second primer. Specifically, the first primer may be added at 0.1 to 1 mol%, 0.1 to 3 mol%, 0.1 to 5 mol%, 0.1 to 7 mol%, 0.1 to 10 mol%, 1 to 3 mol%, 1 to 5 mol%, 1 to 7 mol%, 1 to 10 mol%, 3 to 5 mol%, 3 to 7 mol%, 3 to 10 mol%, 5 to 7 mol%, 5 to 10 mol% or 7 to 10 mol% compared to the second primer.

According to one aspect, by adjusting the addition ratio of the first primer and the second primer, the efficiency of the nucleic acid amplification method can be increased, the target nucleic acid for each sample can be recovered in a constant amount in the selection step, and the sequencing reads for each sample can be consistently adjusted during the NGS library manufacturing stage. Additionally, the ratio can be adjusted differently depending on the type of the second base sequence.

Still another aspect provides a kit for nucleic acid amplification comprising the first primer and the second primer, wherein
the first primer is a polynucleotide consisting of a base sequence including a first base sequence that hybridizes to the target nucleic acid; and
a fixed second base sequence that distinguishes the sample from which the target nucleic acid is derived, and
the second primer is a polynucleotide consisting of a base sequence including a third base sequence that hybridizes to the target nucleic acid.

The terms "first primer", "second primer", "target nucleic acid", "sample", "first base sequence", "second base sequence" and "third base sequence" may be within the aforementioned range.

According to one aspect, when performing the nucleic acid amplification method with the above kit, target nucleic acids derived from different samples can be distinguished.

In one aspect, in the kit,
the first primers may be plural and different from each other,
the second primers may be plural and different from each other, and
the first primers, which are plural and different from each other, may be polynucleotides composed of base sequences including the second base sequences, which are fixed and different from each other.

According to one aspect, the first primer in the above kit may be added at 0.1 to 10 mol% compared to the second primer. Specifically, the first primer may be added at 0.1 to 1 mol%, 0.1 to 3 mol%, 0.1 to 5 mol%, 0.1 to 7 mol%, 0.1 to 10 mol%, 1 to 3 mol%, 1 to 5 mol%, 1 to 7 mol%, 1 to 10 mol%, 3 to 5 mol%, 3 to 7 mol%, 3 to 10 mol%, 5 to 7 mol%, 5 to 10 mol% or 7 to 10 mol% compared to the second primer.

According to one aspect, by adjusting the addition ratio of the first primer and the second primer, the efficiency of the nucleic acid amplification method can be increased, the target nucleic acid for each sample can be recovered in a constant amount in the selection step, and the sequencing reads for each sample can be consistently adjusted during the NGS library manufacturing stage. Additionally, the ratio can be adjusted differently depending on the type of the second base sequence.

In one aspect, the kit may further include a well, and the first primer and the second primer may be seeded into the well. Specifically, the well may be one or more selected from the group consisting of a 8-strip tube, a 96-well plate and a 384-well plate.

In one aspect, the first primer and the second primer may be freeze-dried. If the first primer and the second primer are freeze-dried, deterioration can be prevented.

In one aspect, the first primer and the second primer may be provided bound to beads with a mass of 1 ng to 1 µg or beads with a diameter of 1 um to 10 mm. Due to the binding of the beads, the nucleic acid amplification method can be performed by dividing primers with different barcode sequences into wells or droplets without pipetting or dispensing. The beads may be made of polystyrene or hydrogel. Additionally, the primer may be provided covalently bound to the bead surface, or may be provided entrapped in a gel.

In one aspect, the above kit can be used to perform a nucleic acid amplification method in which more than one strand displacement reaction occurs.

### [Advantageous Effects]

According to the nucleic acid amplification method according to one aspect, which comprises the step of amplifying nucleic acids by adding a first primer, a second primer and a nucleic acid polymerase to a sample containing a target nucleic acid, by performing parallel multiple displacement amplification using the first primer containing the barcode sequence, the increased parallelism and throughput of MDA dramatically reduces the time and cost for NGS library production. Further, the method can also resolve the barcode bias phenomenon by adjusting the ratio of primers containing the barcode sequence and primers that do not contain the barcode sequence, and the quality of amplification can also be improved using a laser-based cell separation method. Therefore, the nucleic acid amplification method can be usefully used for large-scale single-cell whole genome analysis.

### [Description of Drawings]

Fig. 1 shows the genome coverage value in the amplification method using MDA and Tn5 transposase.
Fig. 2A shows the structure of a barcode-containing primer for barcode multiple displacement amplification (bMDA).
Fig. 2B shows the structure of a barcode-containing primer for barcode multiple displacement amplification (bMDA) and a typical N6 primer.
Fig. 2C shows a flow chart schematically depicting the entire process of bMDA.
Fig. 3 shows a flow chart showing the process of performing the NGS library preparation process in one-pot by pooling amplification products derived from different target samples (cells) after the bMDA reaction.
Fig. 4A shows the structure of the R15B8N6 primer consisting of N6, barcode sequence region, and Read 1 (15-mer nucleotide).
Fig. 4B shows that when a primer (R15B8N6) containing an additional sequence other than the N6 primer is used, the MDA reaction is significantly inhibited.
Fig. 4C shows that Read 1 in the R15B8N6 primer acts as a handle for PCR, enabling selective enrichment of barcoded DNA.
Fig. 5 indicates that high concentration of primer is one of the causes of inhibition of MDA reaction.
Fig. 6 indicates that the long length of the primer is one of the causes of MDA reaction inhibition.
Fig. 7 shows the results of a quantitative analysis of the relationship between the concentration and length of the barcode-containing primer and the inhibition of bMDA reaction.
Fig. 8A shows a model in which a barcode-containing primer binds to DNA polymerase and inhibits DNA polymerase, thereby reducing bMDA amplification efficiency.
Fig. 8B shows a model in which a barcode-containing primer is non-specifically hybridized with another primer and forms a primer dimer, thereby reducing MDA amplification efficiency.
Fig. 9 shows the change in amplification efficiency of bMDA measured by varying the amount of phi29 DNA polymerase.
Fig. 10 shows the calculated and experimental values of library complexity according to the bMDA reaction results.
Fig. 11 shows that genome coverage, amplification uniformity, Allele Drop Out (ADO), and false positive rate (FPR) measured after bMDA-seq are similar to conventional MDA.
Fig. 12 shows data comparing the cost of cell processing and genome analysis using MDA with the cost of cell processing and genome analysis using bMDA.
Fig. 13A shows a structure in which biotin is bound as a capture material to the 5' end of the barcode-containing primer, and that this can prevent ligation between the R1 sequence of the adapter and the barcode sequence.
Fig. 13B shows that the location of the bMDA barcode sequence is mainly found in Read 2.
Fig. 14 shows the composition of the barcode found in NGS results after bMDA-seq.
Fig. 15 shows the barcode swapping rate after bMDA-seq.
Fig. 16 shows the amount of barcoded DNA (barcoded product mass) contained in the bMDA amplification product according to 16 different barcode sequences, and the NGS library conversion efficiency (library conversion yield).
Fig. 17 shows the change in the number of NGS reads according to the ratio of primers containing barcode in the bMDA reaction solution.
Fig. 18 shows the degree of barcode bias when the ratios of barcode-containing primers are all set to a constant ratio and when each ratio is set differently.
Fig. 19 shows the CV value of the number of NGS reads according to the change in the amount of template used as input to bMDA.
Fig. 20 shows a chart comparing the differences between fluid-based single-cell separation technology and laser-based single-cell separation technology when applied to bMDA.
Fig. 21 shows the increase in amplification throughput of bMDA compared to MDA and the increase in amplification quality of the laser-based separation method compared to the fluid separation method.
Fig. 22 shows the cost reduction effect that occurs when using bMDA and laser-based separation methods.

### [Best Mode]

Hereinafter, the present invention will be described in detail by Examples. However, the following Example is intended to illustrate the present invention, and the content of the present invention is not limited to the Example.

### Example

### Example 1. Method for performing bMDA-seq

### (1) Cell lysis step

bMDA refers to the MDA reaction using a barcode-containing primer, and bMDA-seq refers to the entire process of preparing an NGS library from a single-cell containing bMDA. The first step for bMDA-seq is to separate and lyse cells. For this, 7 µL of lysis solution (3 µL denaturation buffer(400 mM KOH, 10 mM EDTA and 100 mM DTT), 2 µL PBS (REPLI-g Single Cell Kit, Qiagen), 1 µL template, and 1 µL 500 µM random hexamer) was prepared and reacted at 4°C or lower for 10 to 30 minutes. The template refers to a solution containing target samples such as cells and gDNA. Lysis at low temperature has the advantage of causing much less spontaneous DNA damage such as cytosine deamination and thus fewer DNA errors. Afterwards, 3 µL of neutralization buffer (400 mM HCl and 600 mM Tris-HCl (pH 7.5)) was added and mixed.

### (2) bMDA step

### 1) Summary

In order to sequence DNA derived from multiple cells at once through the library preparation process, a method of performing MDA using a barcode-containing primer was designed (bMDA) .

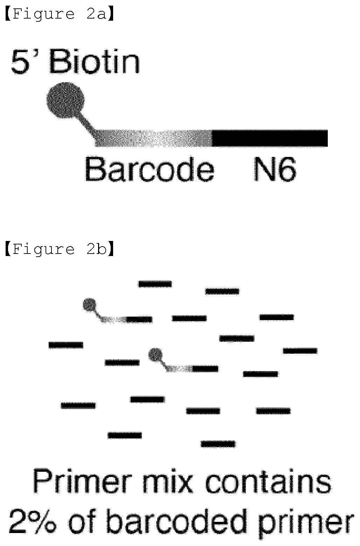

The barcode-containing primer may be composed of a binding region, a barcode sequence region, and capture material (see Fig. 2a). For example, the above primer containing a above barcode may be structured as follows:

/5Biosg/JJJJJJNNNN*N*N.

(Here, /5Biosg/ is a 5'-biotin modification and is a capture material, JJJJJJ is a barcode sequence region composed of 6 bases, NNNNNN is a binding region composed of 6 random bases, and * is a phosphorothiolate modified nucleotide that is resistant to 3'-5'nucleic acid exonuclease reaction.)

The binding region is a sequence that initiates amplification by hybridization with the target sample, and a random hexamer (N6) consisting of 6 random nucleotides was used. To prevent primer depletion due to the 3'-5'nucleic acid exonuclease function of DNA polymerase, the bonds between some nucleotides in the random hexamer were modified to phosphorothiolate bonds.

The barcode sequence is a sequence to distinguish DNA derived from different samples, and a sequence consisting of 4 to 12 nucleotides was used. For example, when using a barcode sequence consisting of 6 nucleotides, by designing 4⁶ = 1,024 different barcodes, 1,024 cells can be analyzed in parallel (multiplexing) at once (one-pot).

The capture material is a material for selectively capturing only barcoded amplification products, and biotin or a polynucleotide consisting of 10 or more base sequences was used. When biotin was used as the capture material, IDT's 5'Biotin modified oligonucleotide was used. When using a polynucleotide consisting of 10 or more base sequences as a capture material, for compatibility with Illumina's sequencer, some base sequences from the 3' terminal region of the Read 1 sequence are borrowed and ACGCTCTTCCGATCT (SEQ ID NO: 1 ) sequence was used.

Biotin has the property of binding strongly to streptavidin, so barcoded DNA can be selected using it. The polynucleotide consisting of 10 or more base sequences can act as a handle for PCR and selectively enrich barcoded DNA.

In order to minimize the inhibition of MDA reaction caused by the above primers containing a barcode, experiments were conducted by mixing regular random hexamers (N6) and modified primers at a specific ratio, and it was confirmed that a primer mix containing 2% modified primers was found to be the most effective. This was confirmed (see Fig. 2B).

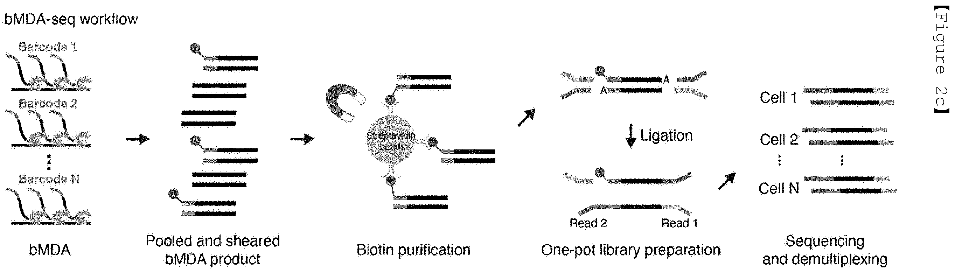

When performing MDA using the above primer containing a above barcode (bMDA), a primer with a random hexamer sequence complementary to the base sequence of the target sample is hybridized to the target sample, and the barcode sequence within the primer is amplified together with the target sample. Therefore, by performing bMDA on different target samples using primers containing different barcodes, pooling the amplification products, collecting them all at once, and then fragmenting them into short lengths, it is possible to obtain amplification products that contain the barcode sequence and those that do not. At this time, only DNA fragments to which biotin is bound, that is, containing the barcode, can be selected using streptavidin coated magnetic beads. After the streptavidin-biotin purification process, a library preparation process is performed, and then NGS (Next Generation Sequencing) is performed to confirm the barcode sequence, a demultiplexing process is possible to determine which sample the corresponding NGS read originated from (see Fig. 2C).

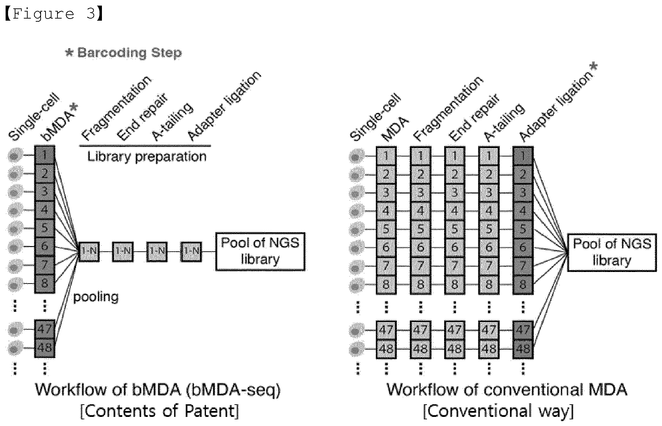

The bMDA, unlike existing MDA, has the feature that it can be parallelized with little change to the existing workflow by directly performing DNA barcoding on the target sample during the MDA amplification process. By pooling amplification products derived from different target samples after bMDA and performing the NGS library preparation process at once, the library preparation cost library preparation cost can be innovatively reduced to 1/N (N = number of samples or barcodes analyzed at once) (see Fig. 3).

### 2) Identifying causes that inhibit the efficiency of bMDA

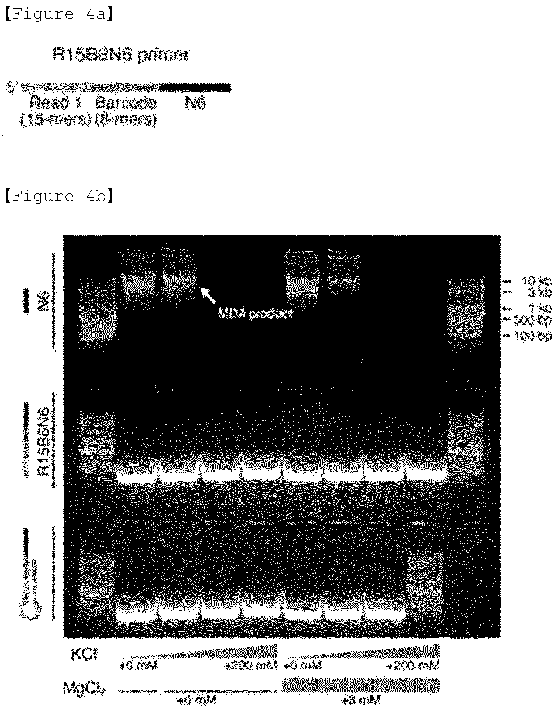

However, if a primer containing an additional sequence other than the N6 primer generally used in the MDA process is used, the MDA reaction is significantly inhibited. To confirm this, MDA was performed by adding 50 µM of N6 primer and 50 µM of R15B8N6 primer, respectively, and the amplification product was subjected to electrophoresis. The R15B8N6 primer consists of a random hexamer (N6) as a binding region, an 8-mer (B8) as a barcode sequence region, and a 15-mer nucleotide (R15) as a capture material (see Fig. 4A). As the R15 sequence, the ACGCCTTCCGATCT (SEQ ID NO: 1) sequence, which is part of Illumina's Read 1 sequence, was used. As a result of electrophoresis, when the N6 primer was used, the amplification product appeared to be a typical length of several to tens of kb, confirming that MDA amplification was successful. However, when 50 µM of R15B8N6 was used, it was confirmed that no amplification occurred at all (See Fig. 4B).

In order to solve the problem of inhibition of bMDA reaction as described above, two experiments were first performed to determine the cause of this phenomenon.

### ① Primer concentration

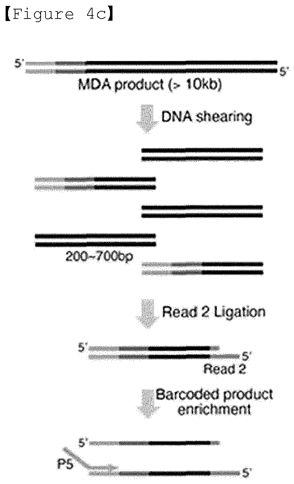

To determine whether the concentration of primers inhibits the bMDA reaction efficiency, MDA was performed by adding 50 µM of N6 primer, 5 µM of N6 primer and 5 µM of R15B8N6 primer. When MDA is performed using a fixed DNA sequence consisting of 15 nucleotides (R15) as a capture material, barcoded DNA can be selectively concentrated by performing PCR using a primer containing the same sequence as R15 (PCR primer) (see Fig. 4C). In the PCR process, DNA that is not barcoded is not subjected to PCR amplification because it does not contain a fixed DNA sequence.

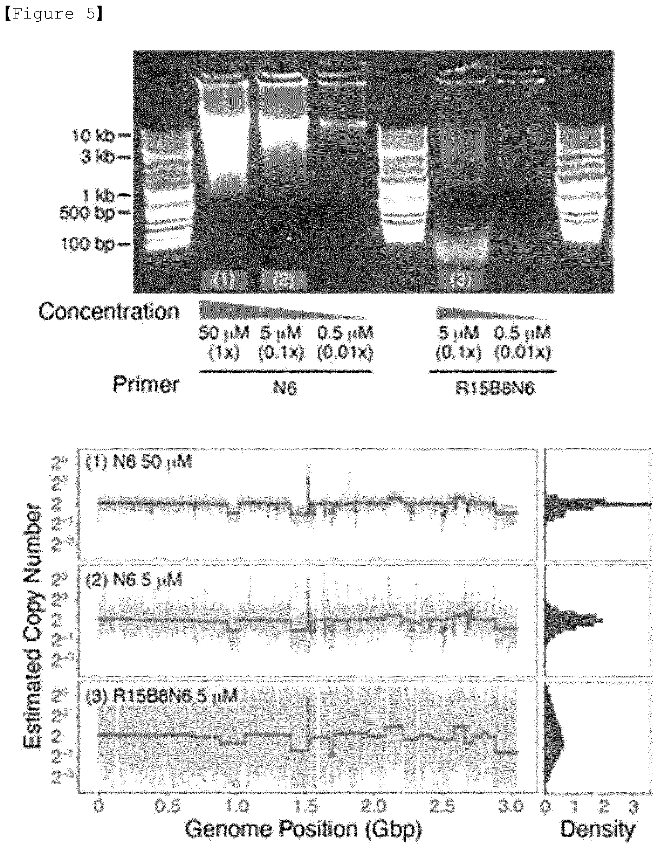

As a result of the experiment, when 5 µM of R15B8N6 primer was added, a somewhat successful amplification product was observed, although the yield was lower than that of 5 µM of N6 (see Fig. 5). This means that a high concentration of R15B8N6 primer is one of the causes of the bMDA reaction inhibition phenomenon. However, to confirm the amplification uniformity of the above experimental results, gene copy number variation (CNA) analysis was performed according to the location of the human genome. As a result, when MDA was performed using 5 µM R15B8N6 primer, the amplification bias (bias) was very severe compared to when 5 µM N6 was used, making it difficult to perform CNA analysis normally (see Fig. 5). In the Estimated Copy Number analysis results in Figure 5, the gray dots show how many NGS sequences are found depending on the location of the human genome. The lower the amplification bias, the more uniformly the genome is amplified, the lower the variation between gray dots. When 5 µM of the R15B8N6 primer is used, it can be seen that the variation between gray dots is very severe. Through this, it can be confirmed that there are other causes that inhibit MDA amplification in addition to the concentration of the R15B8N6 primer.

### ② Primer length

To determine whether the length of the primer inhibits the bMDA reaction efficiency, MDA was performed by adding 50 µM of N6 primer, 50 µM of R15B8N6 primer, 50 µM of N6 primer + 50 µM of R15B8N6 primer, and 500 µM of N6 primer.

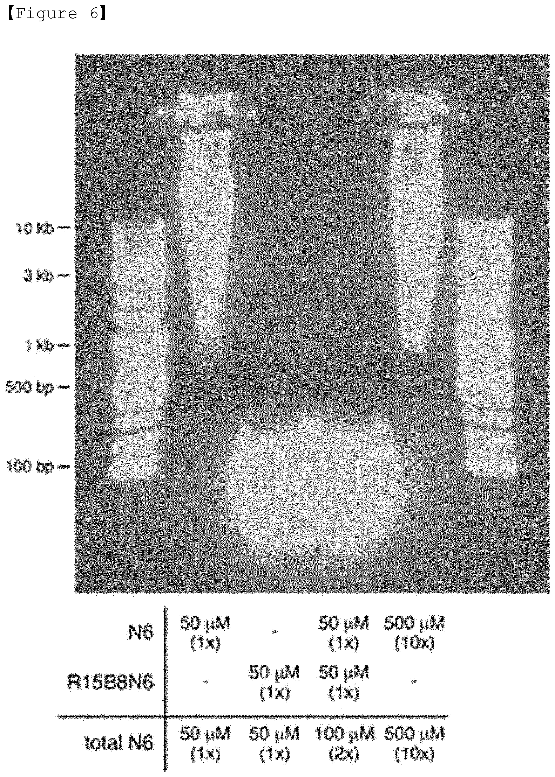

As a result, MDA amplification products were obtained in the group treated with N6 primer 500 µM, which is about 10 times higher than the primer concentration generally used in MDA, but when 50 µM of R15B8N6 was added to the group treated with 50 µM of N6 primer, which is the primer concentration generally used for MDA, MDA was significantly inhibited (see Fig. 6). The R15B8N6 primer is a 29-mer in length, which is about 5 times longer than the N6 primer. Therefore, if the length of the primer does not affect the reaction, adding 50 µM of the R15B8N6 primer should have a similar effect as adding 250 µM of the N6 primer. However, the experimental results show that when adding 50 µM of R15B8N6 primer to 50 µM of N6 primer, MDA amplification is significantly inhibited, but MDA amplification is performed normally even when the N6 primer is increased to 500 µM to contain more nucleic acids than 50 µM of R15B8N6. This means that the long length of the R15B8N6 primer is the cause of the bMDA reaction inhibition phenomenon.

### ③ Quantitative analysis of the relationship between primer concentration and length and bMDA reaction inhibition phenomenon

Additionally, in order to quantitatively analyze the relationship between the concentration and length of the barcode-containing primer and the MDA reaction inhibition phenomenon, the bMDA amplification efficiency was analyzed by varying the length and concentration of the barcode sequence.

The bMDA amplification efficiency was measured by observing an increase in fluorescence in real time by adding SYTO^{™} 13(Invitrogen), which reacts with double-stranded DNA (dsDNA) and specifically fluoresces during the bMDA reaction(RT-MDA, Real time MDA), and the maximum slope of the increase in fluorescence intensity was defined as amplification efficiency. For comparison, the amplification efficiency value was divided by the amplification efficiency value of a typical MDA using random hexamers and normalized so that the maximum value was 1. To reduce changes in primer length, bMDA reaction was performed using barcode-containing primers of various lengths. Specifically, primers consisting of 29-mer(ACGCTCTTCCGATCTGCCAAGACNNNN*N*N, SEQ ID NO: 2), 14-mer (GACTGTGTNNNN*N*N, SEQ ID NO: 3), 13-mer(ACTGTGTNNNN*N*N, SEQ ID NO: 4), 12-mer (CTGTGTNNNN*N*N, SEQ ID NO: 5), 11-mer(TGTGTNNNN*N*N, SEQ ID NO: 6) and 10-mer(GTGTNNNN*N*N, SEQ ID NO: 7) were used. At this time, the * refers to phosphorothiolate modification. When increasing or decreasing the concentration of barcode-containing primers, the same amount of random hexamers was decreased or increased to keep the total amount of primers including random hexamers constant.

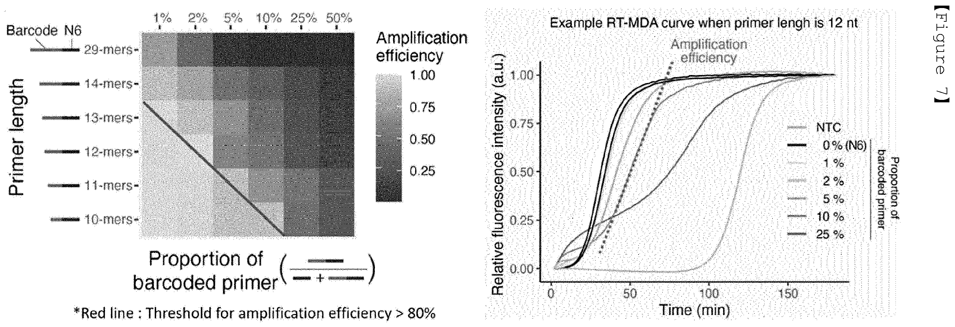

As a result of the analysis, it was confirmed that as the proportion of barcoded primers increases and the length of the primers increases, the bMDA amplification efficiency decreases dramatically (see Fig. 7).

### ④ Analysis of the cause of inhibition of bMDA reaction depending on primer concentration and length

As a cause of inhibition of bMDA reaction depending on primer concentration and length, the following two models can be considered.

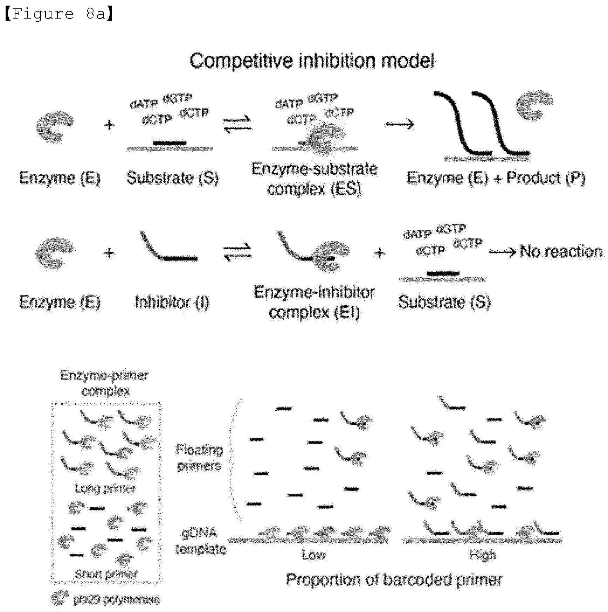

First, a barcode-containing primer binds to DNA polymerase and acts as an inhibitor of DNA polymerase (competitive inhibition model, see Fig. 8A). All DNA polymerases basically have the ability to bind DNA, and as the length of the primer increases, the binding affinity between the primer and DNA polymerase increases. Therefore, the longer the barcode sequence, the more DNA polymerase binds to the barcode-containing primer, which may lead to more depletion of DNA polymerase, resulting in lower bMDA amplification efficiency.

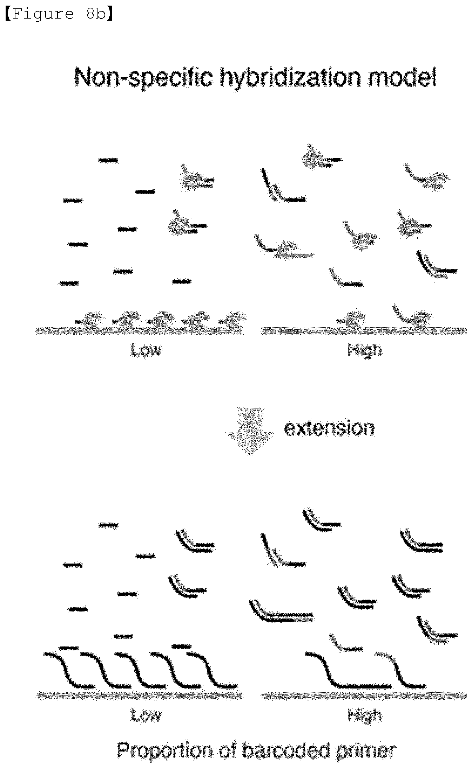

Second, there is a case where a barcode-containing primer hybridizes non-specifically with another primer (see Fig. 8B). In this case, primer dimers may be formed, reducing bMDA amplification efficiency.

Through the above experiments, it was confirmed that when bMDA is performed with the barcode-containing primer, amplification efficiency decreases, and that the concentration and length of the primer have a significant effect on MDA amplification efficiency. As a way to increase the efficiency of bMDA by minimizing the inhibition of bMDA amplification efficiency by primers containing barcode, the following five methods can be considered.

### 3) Identify ways to increase the efficiency of bMDA

### ① Method for adding excessive amounts of DNA polymerase

First, there is a method of adding excessive amounts of DNA polymerase. This is because, as mentioned above, as the barcode sequence becomes longer, the binding affinity between the barcode-containing primer and DNA polymerase increases, resulting in greater depletion of DNA polymerase. For example, for MDA, an amount of enzyme capable of polymerizing about 100 pmole of dNTP per minute at 30°C is usually added, and an amount of enzyme capable of polymerizing about 1 nmole or more of dNTP per minute at 30°C is added. By adding it, it is possible to prevent a decrease in the amplification efficiency of bMDA by offsetting enzyme depletion caused by the barcode-containing primer.

To verify the above idea, bMDA was performed with different amounts of DNA polymerase (phi29 DNA polymerase) and the efficiency was measured. The bMDA reaction efficiency value according to the ratio of the amount of DNA polymerase and the barcode-containing primer was measured using the RT-MDA technique mentioned above, and normalization was performed so that the maximum reaction efficiency value of all data was 1. As the barcode-containing primer, /5Biosg/ACTCTGNNNN*N*N (SEQ ID NO: 8) was used.

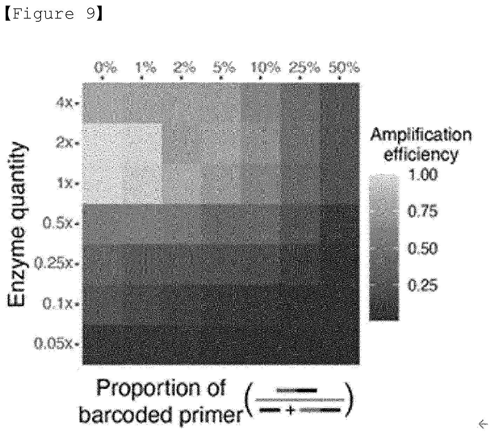

As a result, it was confirmed that even if the amount of DNA polymerase was increased, the decline in amplification efficiency could not be completely prevented (see Fig. 9). Specifically, when the amount of DNA polymerase (Enzyme quantity) is 1x and the ratio of primers containing barcode is 1%, the bMDA amplification efficiency shows a value of 0.93, and when the amount of polymerase was increased by 2x, the value was 0.96, and when it was increased by 4x, the value was 0.78. This means that if the amount of DNA polymerase is increased to 2x, the amplification efficiency can be slightly improved, but if the amount of DNA polymerase is increased to 4x, the amplification efficiency decreases again. The above phenomenon is presumed to be caused by the effect of enzyme storage buffer added to the bMDA reaction solution to increase the amount of DNA polymerase.

### ② Way to reduce the amount of barcode-containing primer

The second method is to reduce the amount of the barcode-containing primer during bMDA reaction. However, even if this reduces the amount of primer that binds to DNA polymerase (or the formation of primer dimers), when the concentration of primers decreases in the MDA reaction, the problem of lowering amplification uniformity occurs (see Fig. 5). This is because the more strand displacement that occurs during the MDA reaction, the higher the MDA amplification speed and amplification uniformity. For this reason, in a typical MDA reaction, the primer concentration is maintained at a high concentration of 10 µM or more. Therefore, this is likely not an appropriate solution.

### ③ Way to mix barcode-containing primers and primers without barcodes at a specific ratio

The third method is to perform bMDA by mixing a barcode-containing primer and a primer that does not contain a barcode and only has a binding region (for example, a random hexamer or common base) at a specific ratio. The above method prevents a decrease in bMDA amplification efficiency by reducing the amount of barcode-containing primer, while maintaining amplification uniformity by maintaining the concentration of the primer containing N6 required for the MDA reaction at a similar high level as before. Since the barcode-containing primer contains N6, the concentration of the primer containing N6 means the sum of the concentration of the barcode-containing primer and the concentration of the N6 primer commonly used in MDA.

### ④ Way to shorten the length of barcode-containing primers

According to the above experimental results showing that the longer the length of the primer, the lower the bMDA amplification efficiency (see Fig. 7), for efficient bMDA, it is desirable to have no additional nucleic acid sequences in the primer other than the barcode sequence for parallelization and the N6 sequence to initiate bMDA amplification. Therefore, if a polynucleotide consisting of more than 10 base sequences is used as a capture material in the barcode-containing primer, bMDA efficiency will be impaired. Therefore, a method of selectively concentrating only barcoded DNA using biochemical molecules such as biotin can be a very effective method to solve the problem of decreased bMDA efficiency. Additionally, as the length of the barcode sequence decreases, bMDA amplification efficiency will increase.

### ⑤ Way to appropriately adjust the length and ratio of barcode-containing primers

### i) Determination of appropriate length and ratio of barcode-containing primers

In order to increase bMDA amplification efficiency, it is desirable to reduce both the length and concentration of the barcode-containing primer as much as possible, while in order for bMDA to function normally, it is necessary to increase both the length and concentration of the primer as much as possible.

First, the barcode length must be long enough to increase the barcoding capacity that can be parallelized at once. Here, barcode length means the total length of the barcode-containing primer minus 6, which corresponds to the N6 length.

Second, the ratio of barcode-containing primers must be sufficiently high to increase library complexity. Library complexity is a value that indicates how high a depth of coverage the human genome can be analyzed when only barcoded amplification products are collected from bMDA amplification products. If the ratio of barcode-containing primers is too low, the amount of DNA fragments containing the barcode recovered will be small, and the NGS sequencing result will not sufficiently cover the human genome. Therefore, single-cell genome analysis at the single base sequence level will become impossible.

In summary, in order to increase bMDA amplification performance and amplification uniformity, the length and ratio of the barcode-containing primer must be reduced. However, as the length of the barcode is reduced, the barcode capacity decreases, and as the ratio of barcode-containing primers is reduced, the amount of barcoded amplification product in the bMDA amplification product decreases, thereby reducing library complexity. Therefore, it is essential for technicians to make efforts to use primers with barcode sequences of appropriate lengths in appropriate ratio while maintaining high bMDA amplification efficiency.

In order to find the appropriate ratio of barcode-containing primers, we first calculated the theoretical library complexity when the barcode-containing primers were present at a ratio of 2% in the total primers. Typically, bMDA amplification products have a length distribution of about 10 kbp, and when DNA is fragmented to have an average length of about 200 bp, the DNA fragments containing the barcode among the bMDA amplification products are calculated to be 0.04% of the total (barcode-containing primer ratio 2% * 200 bp / 10 kbp = 0.04 %). In addition, since the efficiency of library production is usually about 20 to 40%, assuming the minimum value of 20%, the proportion of DNA that is ultimately converted to NGS library among bMDA amplification products is calculated to be 0.008% (DNA fragment containing barcode 0.04% * library conversion efficiency 20% = 0.008 %).

To experimentally measure the ratio of DNA fragments containing the barcode (biotinylated dsDNA fragment ratio), the DNA weight of the bMDA amplification product and the DNA weight after streptavidin-biotin purification were measured and divided. In addition, to measure library conversion efficiency (NGS library conversion rate), DNA weight was measured before and after library preparation, and it was assumed that PCR for library amplification increases the amount of DNA by 1.9 times per cycle (PCR cycle).

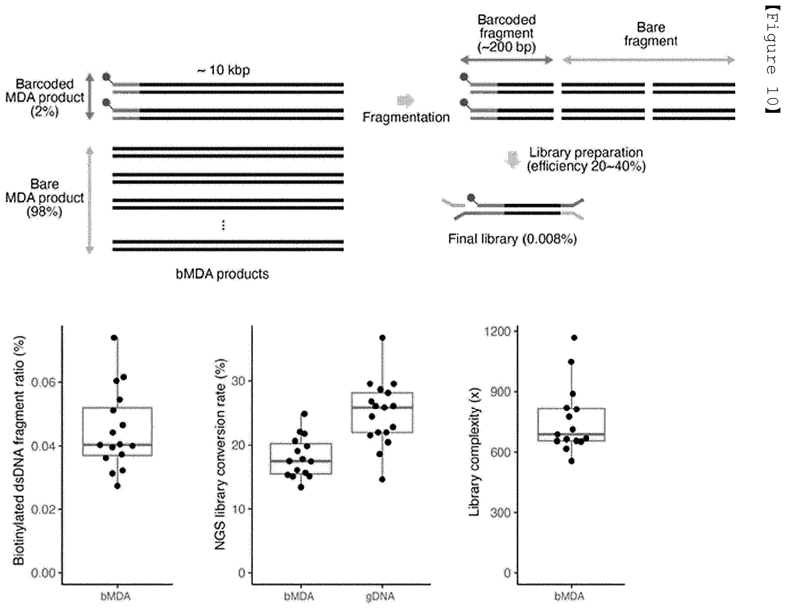

As a result of measurement through actual experiments, it was confirmed that the ratio of barcode-containing DNA fragments (Biotinylated dsDNA fragment ratio) was 0.04% on average, and the library conversion efficiency (NGS library conversion rate) was approximately 20% (see Fig. 10).

Typically, since a large amount of amplification product of more than 30 µg can be obtained through MDA reaction, about 2.4 ng of DNA out of 30 µg of bMDA reaction product is converted into NGS library(30 µg * NGS library conversion ratio of bMDA 0.008 % = 2.4 ng). This corresponds to approximately 727x of the total human genome (2.4 ng / 3.3 pg (weight of human genome haploid) = 727), meaning that the human genome can be read up to 727 times using the NGS library obtained from bMDA(Library complexity = 727x). Typically, considering that more than 10x library complexity is required for single-cell genome analysis at the single base sequence level, the above results mean that even if the barcode-containing primer is added at a low rate of about 2%, the human genome is sufficiently covered and there is no problem in genome analysis at the single-cell level.

The ratio of barcode-containing primers is 2%, and the length of the barcode that maintains bMDA amplification efficiency of more than 80% appears to be 6-mer or less(see Fig. 7 and Table 1). When the barcode length is set to 6-mer, the barcode capacity is 4,096, which is judged to be sufficiently high for the performance of bMDA for the purpose of single-cell genome analysis at the single base sequence level, so the barcode length of 6-mer and the ratio of barcode-containing primers of 2% were determined as the bMDA experiment conditions.

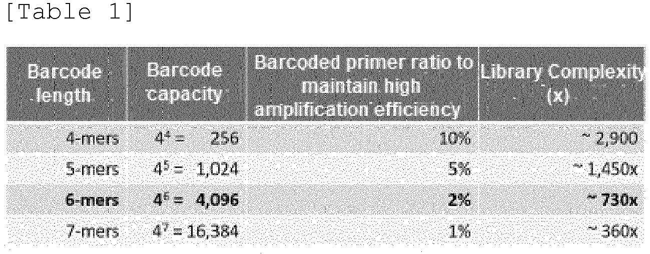

### ii) Analysis of bMDA results with 2% of 6-mer length barcode-containing primer added

bMDA was performed on a single cell under the following conditions under the condition that 2% of the 6-mer length barcode-containing primer was added compared to the total primer, and then sequencing was performed using NGS. Afterwards, the differences between conventional MDA and bMDA were measured in various aspects such as genome coverage, mean depth of coverage, amplification uniformity (coverage uniformity), gene copy number alteration (CNA) analysis, and single nucleotide variation (SNV) analysis.

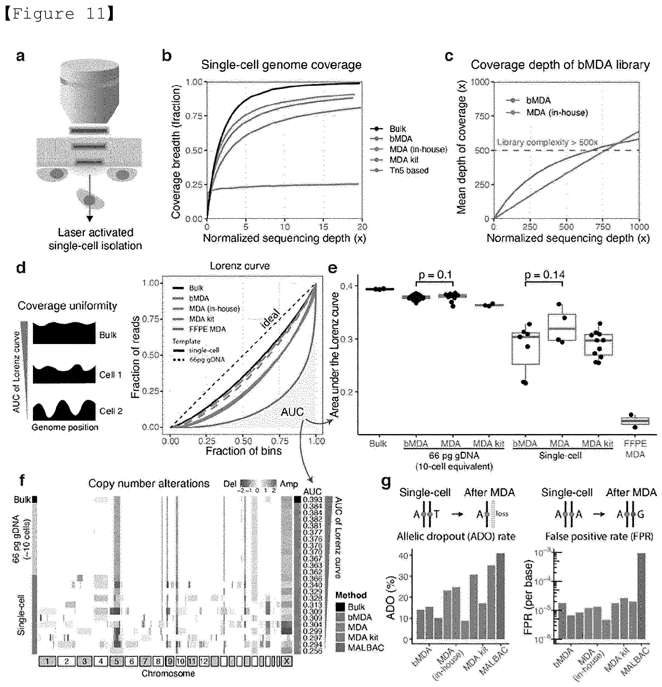

First, to isolate single cells, HL-60 cells were smeared on a 100 nm thick ITO-coated glass slide. The ITO is used for the purpose of selectively separating cells in the laser-irradiated area by absorbing light and vaporizing it when an infrared laser is irradiated. While observing the slide glass on which HL-60 cells were spread through a microscope image, an infrared laser was irradiated to the location where the single-cell was present to separate the single-cell(see Fig. 11A).

The separated single-cell was lysed in the same manner as in Example (1) to obtain a cell lysate (10 µL), and then 40 µL of master mix was added to the lysate. The master mix consisted of 23 µL of water, 5 µL of 10X phi29 DNA polymerase buffer (500 mM Tris-HCl, 100 mM MgCl₂, 100 mM (NH₄)₂SO₄, 40 mM DTT, pH 7.5), 4 µL of dNTPs of 25 mM each, 2 µL of 1 mM random hexamer, 2 µL of phi29 DNA polymerase (Genomiphi V2 DNA amplification kit, Cytiva, cat. no. 25-6600-31), 3.2 µL of 40% (w/v) PEG 8000, 0.25 µL of 1 M DTT, 0.5 µL of 50 µM SYTOTM 13 Green Fluorescent Nucleic Acid Stain (Invitrogen) and 0.05 µL of 500 nM. Meanwhile, for the bMDA experiment, the barcode-containing primer was used at the same concentration instead of the random hexamer, or the barcode-containing primer and the random hexamer were mixed to ensure that the total primer concentration was the same.

For each single-cell isolated as above, bMDA amplification products were obtained using barcode-containing primers with different barcode sequences, and NGS sequencing was performed according to the bMDA amplification product processing steps mentioned below.

In addition, for comparison, experiments were conducted using three methods: a method using a conventional MDA kit (MDA kit), a method using a conventional random hexamer only as a primer under the same MDA experimental conditions as bMDA (in-house MDA), and a method of directly tagging the gDNA of a single-cell using Tn5 transposase and then performing single-cell genome analysis (Tn5 based). The single-cell analysis method using Tn5 was performed according to the protocol in the paper [Xi, L., Leong, P. & Mihajlovic, A. Preparing Single-cell DNA Library Using Nextera for Detection of CNV. Bio-protocol 9, (2019)].

As a result of measuring the genome coverage value according to the above experiment, bMDA showed a value of 86.7 ± 0.97%, a sufficiently high genome coverage to enable genome analysis at the single base sequence level, which is similar to in-house MDA (84.7 ± 3.3% s.e.m.) and MDA kit (81.1%) (see Fig. 11B). The genome coverage (coverage breadth) is a value that indicates what percentage of the entire human genome can be covered when the NGS analysis results are aligned to the reference human genome. The genome coverage value of 86.7% shown by bMDA means that 86.7% of the human genome information of the single-cell subject to amplification can be obtained. This is in contrast to the results of the Tn5-based single-cell genome analysis technology, which showed a genome coverage of 21.6 ± 3.7%. That is, Tn5-based genome analysis technology is unable to analyze the genome at the single base sequence level, but bMDA is capable of analyzing the genome at the single base sequence level.

In addition, as a result of measuring the mean depth of coverage to check how deeply the covered human genome information can be sequenced, it was confirmed that both in-house MDA and bMDA showed an average depth of coverage of 500x or more (Fig. 11C). This means that the bMDA amplification product has a library complexity of 500x or more and is suitable for genome analysis at the single base sequence level. Here, the coverage depth average is a value that indicates how deeply the NGS reads cover the human genome region when the NGS sequencing results are aligned to the human reference genome. For example, the average coverage depth of 500x means that the NGS read covered the human genome about 500 times on average.

Additionally, to measure the coverage uniformity of bMDA and conventional MDA, a Lorenz curve was used to show how uniformly the NGS reads cover the human genome (see Fig. 11D) . In the Lorenz curve, the larger the Area Under Curve (AUC) value, the more uniformly the human genome is covered, which means that the amplification bias due to bMDA or MDA is low. As a result of comparing the AUC values of bMDA and conventional MDA, there was no statistically significant difference between the two groups(see Fig. 11E). When analyzing single-cell, the bMDA group tended to have a slightly lower AUC than the MDA group, but the difference was smaller than the difference within each group (difference between single-cell and single-cell) (one-way ANOVA , p=0.15) was judged to be not significant. In other words, the amplification uniformity of bMDA may be somewhat lower than that of MDA due to the use of barcode-containing primers, but the difference is not practically significant in analyzing single-cells, and it can be concluded that both MDA and bMDA show similar technical performance.

bMDA also showed similar performance to conventional MDA in analyzing gene copy number alteration (CNA) (see Fig. 11f). As a result of performing bMDA on 66 pg of gDNA, all of them provided accurate CNA results, just like in-house MDA or MDA kit(Bulk results and CNA profile are similar). However, when amplification was performed targeting a single-cell, in most cases, except for a few samples with high AUC, accurate CNA analysis was not provided and many false positive and false negative results occurred. However, since both in-house MDA and MDA show similar results, this is interpreted as a fundamental limitation of MDA-based technology rather than a problem caused by the modified primer of bMDA. In fact, when performing MDA analysis from a single-cell, only about 5 to 10% of samples show successful amplification results that enable CNA analysis, and most samples show amplification results that make CNA analysis difficult. This is a limitation of MDA that must be overcome, and the laser-based cell separation technology below is one way to overcome the fundamental limitation of MDA.

Finally, to confirm whether the bMDA technology is suitable for single nucleotide variation (SNV) analysis, ADO (Allele Drop Out) and FPR (False positive rate) values were measured. ADO value refers to the phenomenon in which one of the two alleles present in a single-cell is lost during the MDA amplification process, and FPR refers to the rate at which single nucleotide mutations that did not originally exist occur due to malfunction of DNA polymerase during the MDA amplification process.

As a result of the measurement, bMDA showed similar or better performance than in-house MDA or MDA kit in single nucleotide mutation analysis (see Fig. 11g). Specifically, bMDA showed an ADO value of 13.2 ± 1.6% (s.e.m.; n=3), MDA (in-house) showed an ADO value of 19.0 ± 5.1% (s.e.m.; n=3), and MDA kit showed an ADO value of 27.7 ± 5.4% (s.e.m.; n=3). In addition, bMDA showed an FPR value of 1.1×10⁻⁵ ± 0.35×10⁻⁵ (s.e.m.; n=3), MDA (in-house) showed an FPR value of 0.99×10⁻⁵ ± 0.26×10⁻⁵ (s.e.m.; n=3), and MDA kit showed an FPR value of 2.2×10⁻⁵ ± 0.25×10⁻⁵ (s.e.m.; n=3).

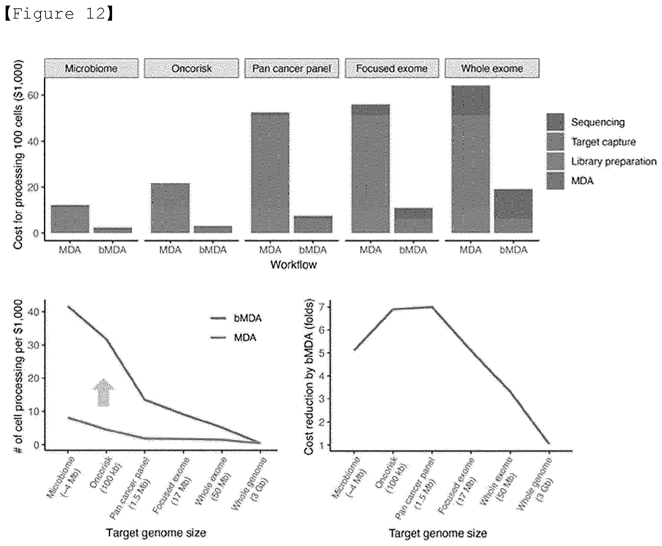

Based on these results, it was confirmed that bMDA performed using the above method has similar performance to conventional MDA or a commercialized MDA kit and that parallelization is possible. As a result, the cost of the library preparation process among the costs of single-cell genome analysis at the single base sequence level can be significantly reduced, reducing the overall cost by about 3 to 7 times (see Fig. 12) .

In conclusion, for bMDA to be parallelizable, the following four requirements must be satisfied: i) Maintaining high primer concentration above 10 pM, ii) Minimization of decline in amplification efficiency by barcode-containing primer, iii) Sufficient length of barcode sequence to barcode large quantities of samples and iv) The amount of barcode-containing primer must be sufficient to obtain a sufficient amount of barcoded amplification product for future sequencing. It was found that mixing barcode-containing primer and random hexamer in an appropriate ratio is the optimal solution that satisfies all four requirements above.

### ⑥ Other ways to increase bMDA efficiency

The amplification efficiency of bMDA can be increased by modifying the enzyme or primer to prevent binding of DNA polymerase and barcode-containing primer. For example, the amplification efficiency of bMDA can be increased by a method of manufacturing the barcode sequence region with RNA, a method of adding a linker between the barcode sequence region and the binding region, a method of combining a physical structure to provide a steric hindrance to the primer, or a method of manipulating DNA polymerase so that it cannot easily bind to ssDNA.

In addition, it is possible to increase the amplification efficiency of bMDA by keeping the concentration of the primer low by configuring the barcode-containing primer to gradually dissolve in the reaction solution during the bMDA reaction.

### (3) bMDA amplification product processing steps

For next generation sequencing (NGS) of the bMDA amplification product, the following processing steps were performed.

### 1) Pooling step

The bMDA amplification products amplified using multiple barcode-containing primers of different types were at an appropriate volume ratio mixed (pooling). Specifically, as a technical verification experiment, a total of 48 different barcode-containing primers were designed, and MDA amplification products were mixed at the same volume ratio.

### 2) DNA first purification step

Solid phase reversible immobilization (SPRI) technique was used to purify DNA from the mixture. Specifically, when the sample volume was 1x, 0.8x sample volume of Ampure magnetic beads were added, DNA was purified according to the protocol, and the purified DNA was eluted in 0.4x volume of water. The eluate was centrifuged at 20,000 g for 1.5 hours to separate the beads from the eluate.

### 3) Fragmentation step

The purified DNA was fragmented to an appropriate length of 50 bp to 10 kb using sonication or enzyme (DNA fragmentase) . The sonication was used to fragment the DNA to a peak length of 200 bp using an S220 Focused-ultrasonicator (Covaris), and then purification was performed once again using SPRI beads.

### 4) Biotin capture and biotin product purification steps

To selectively capture barcoded DNA fragments in bMDA amplification products, Dynabeads MyOne Streptavidin T1 (Invitrogen) beads were used according to the protocol.

However, the binding between streptavidin and biotin is very strong, causing difficulties in the process of separating and recovering DNA after purification. Typically, methods such as high temperature, deionization, acidic conditions, or protein reduction are used to break the bond between streptavidin and biotin. When using such a method, problems arise such as denaturation of dsDNA or greatly reduced recovery rate. In particular, when dsDNA is denatured, the subsequent library preparation process becomes impossible, and when the yield decreases, library complexity is lowered, making single-cell genome analysis at the single base sequence level difficult.

To solve the above problem, an elution buffer containing Sodium Dodecyl Sulfate (SDS) and an excess of D-Biotin (SDS 1%, Tris-HCl (pH 8.0) 10mM, EDTA 1mM, and D-Biotin 0.73mM) was added and incubated at 54°C for 1 hour.

As a result of separating DNA from beads under the above conditions, it was confirmed that DNA could be successfully recovered from streptavidin beads without denaturing dsDNA (see Table 2).

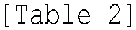

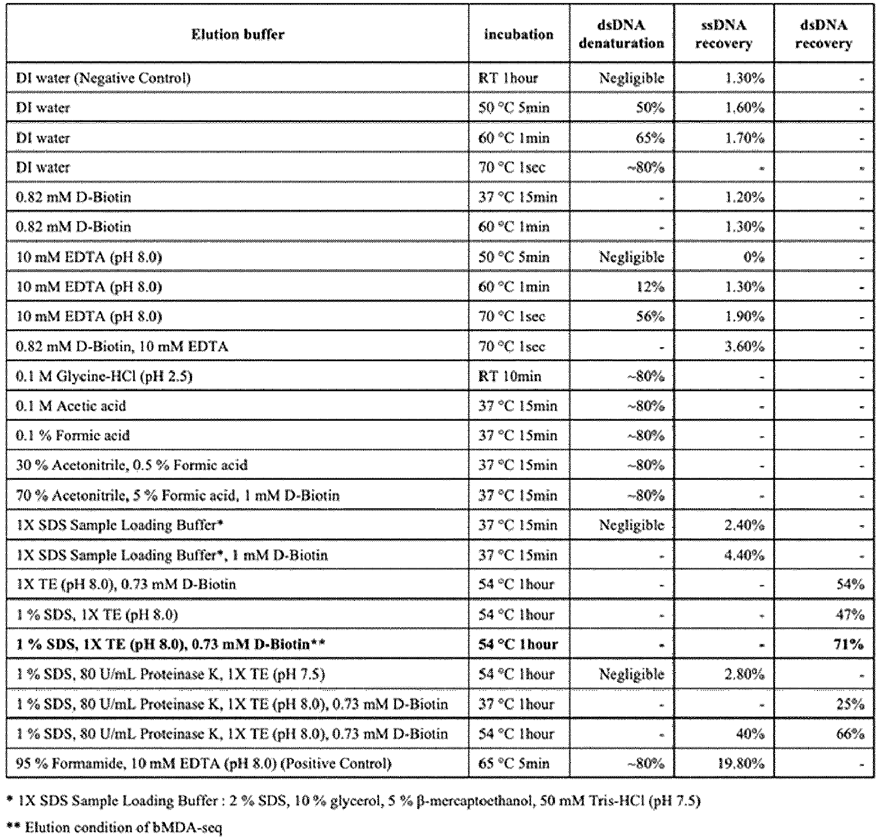

### 5) DNA second purification step

The DNA obtained in step 4) above was purified using MinElute PCR Purification Kit (QIAGEN) or Ampure SPRI beads according to the protocol.

### 6) Library preparation step

Library preparation was performed using the KAPA HyperPrep Kit (KK8500, KAPA Biosystems, Inc., USA). At this time, the incubation for ligation was performed according to the manufacturer's protocol, except that the incubation was extended to 8 hours.

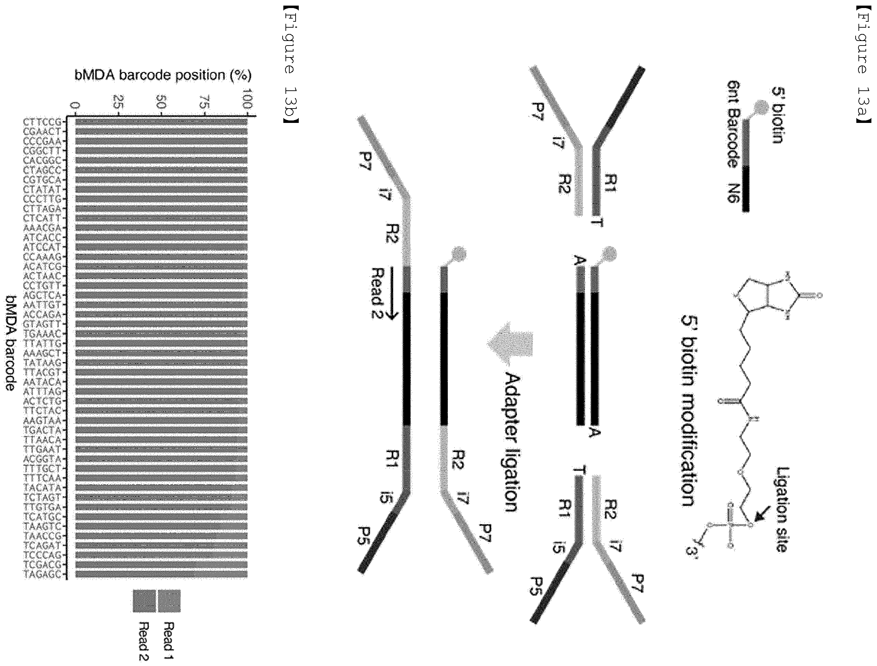

Since biotin is bound to the 5' end of the barcoded DNA, ligation between the R1 strand of the adapter and the biotin-containing DNA strand of the barcoded DNA is prevented during the library preparation process (see Fig. 13A). As a result, only the DNA molecule ligated to the R2 strand of the adapter is amplified, and the barcode is mainly found in Read 2 (see Fig. 13B). However, in the case of barcode sequences starting with TA or TC, the rate found in Read 1 was higher than that of other barcode sequences. This means that how the base sequence of the barcode is designed can be an important factor in bMDA performance. The sequences of 48 barcode-containing primers used in the above experiment are shown in Table 3 below.

(In Table 3, /5Biosg/ is a 5'-biotin modification and is a capture material, NNNNNN is a binding region composed of 6 random bases, and * is a phosphorothiolate modified nucleotide that is resistant to 3'-5' nucleic acid exonuclease reaction.)

**[Table 3]**

| Primer Sequence | |
|---|---|
| /5Biosg/ACTCTGNNNN*N*N(SEQ ID NO: 8) | /5Biosg/CTTCCGNNNN*N*N(SEQ ID NO: 9) |
| /5Biosg/CGAACTNNNN*N*N(SEQ ID NO: 10) | /5Biosg/CCCGAANNNN*N*N(SEQ ID NO: 11) |
| /5Biosg/CGGCTTNNNN*N*N(SEQ ID NO: 12) | /5Biosg/CACGGCNNNN*N*N(SEQ ID NO: 13) |
| /5Biosg/CTAGCCNNNN*N*N(SEQ ID NO: 14) | /5Biosg/CGTGCANNNN*N*N(SEQ ID NO: 15) |
| /5Biosg/CTATATNNNN*N*N(SEQ ID NO: 16) | /5Biosg/CCCTTGNNNN*N*N(SEQ ID NO: 17) |
| /5Biosg/CTTAGANNNN*N*N(SEQ ID NO: 18) | /5Biosg/CTCATTNNNN*N*N(SEQ ID NO: 19) |
| /5Biosg/AAACGANNNN*N*N(SEQ ID NO: 20 | /5Biosg/ATCACCNNNN*N*N(SEQ ID NO: 21) |
| /5Biosg/ATCCATNNNN*N*N(SEQ ID NO: 22) | /5Biosg/CCAAAGNNNN*N*N(SEQ ID NO: 23) |
| /5Biosg/ACATCGNNNN*N*N(SEQ ID NO: 24) | /5Biosg/ACTAACNNNN*N*N(SEQ ID NO: 25) |
| /5Biosg/CCTGTTNNNN*N*N(SEQ ID NO: 26) | /5Biosg/AGCTCANNNN*N*N(SEQ ID NO: 27) |
| /5Biosg/AATTGTNNNN*N*N(SEQ ID NO: 28) | /5Biosg/ACCAGANNNN*N*N(SEQ ID NO: 29) |
| /5Biosg/GTAGTTNNNN*N*N(SEQ ID NO: 30) | /5Biosg/TGAAACNNNN*N*N(SEQ ID NO: 31) |
| /5Biosg/TTATTGNNNN*N*N(SEQ ID NO: 32) | /5Biosg/AAAGCTNNNN*N*N(SEQ ID NO: 33) |
| /5Biosg/TATAAGNNNN*N*N(SEQ ID NO: 34) | /5Biosg/TTACGTNNNN*N*N(SEQ ID NO: 35) |
| /5Biosg/AATACANNNN*N*N(SEQ ID NO: 36) | /5Biosg/ATTTAGNNNN*N*N(SEQ ID NO: 37) |
| /5Biosg/TTCTACNNNN*N*N(SEQ ID NO: 38) | /5Biosg/AAGTAANNNN*N*N(SEQ ID NO: 39) |
| /5Biosg/TGACTANNNN*N*N(SEQ ID NO: 40) | /5Biosg/TTAACANNNN*N*N(SEQ ID NO: 41) |
| /5Biosg/TTGAATNNNN*N*N(SEQ ID NO: 42) | /5Biosg/ACGGTANNNN*N*N(SEQ ID NO: 43) |
| /5Biosg/TTTGCTNNNN*N*N(SEQ ID NO: 44) | /5Biosg/TTTCAANNNN*N*N(SEQ ID NO: 45) |
| /5Biosg/TACATANNNN*N*N(SEQ ID NO: 46) | /5Biosg/TCTAGTNNNN*N*N(SEQ ID NO: 47) |
| /5Biosg/TTGTGANNNN*N*N(SEQ ID NO: 48) | /5Biosg/TCATGCNNNN*N*N(SEQ ID NO: 49) |
| /5Biosg/TAAGTCNNNN*N*N(SEQ ID NO: 50) | /5Biosg/TAACCGNNNN*N*N(SEQ ID NO: 51) |
| /5Biosg/TCAGATNNNN*N*N(SEQ ID NO: 52) | /5Biosg/TCCCAGNNNN*N*N(SEQ ID NO: 53) |
| /5Biosg/TCGACGNNNN*N*N(SEQ ID NO: 54) | /5Biosg/TAGAGCNNNN*N*N(SEQ ID NO: 55) |

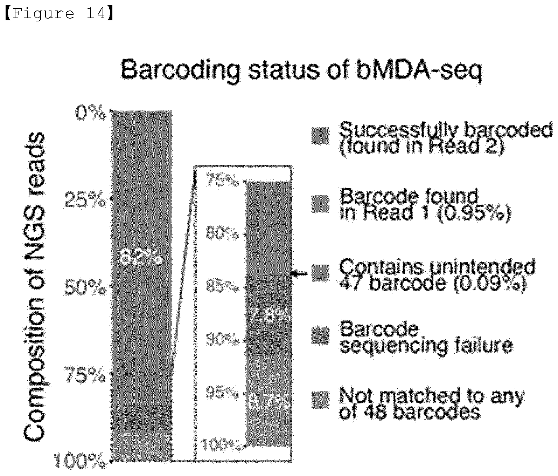

After library preparation, NGS was performed using the Illumina platform, and as expected, barcode sequences were found in most of NGS Read 2 (about 82%) (see Fig. 14). In other words, the first six bases of Read 2 among the NGS results can be viewed as the barcode sequence that exists within the barcode-containing primer. Using this information, the data can be deparallelized (demultiplexed) to separate which cell the NGS sequencing result is from (see Fig. 2C).

Meanwhile, in order to check whether barcode swapping, in which barcodes are reversed, occurs during a series of experiments performed at once by pooling bMDA amplification products containing multiple barcode information, among a total of 48 barcode-containing primers, one primer used gDNA derived from mice (NIH3T3 cell, cat. no. 21658, Korean Cell Line Bank), and for the remaining 47 primers, bMDA was performed using gDNA from human-derived HL-60 cells.

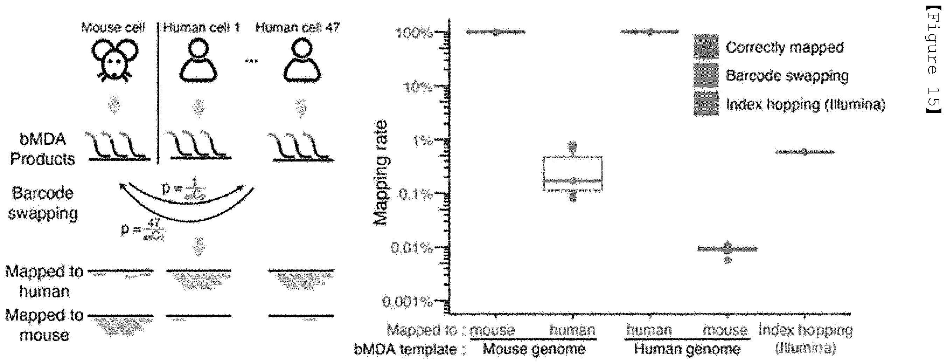

As a result, it was confirmed that when mouse DNA was used as a template, most NGS reads were mapped to the mouse reference genome, and when human DNA was used as a template, most NGS reads were mapped to the human reference genome (see Fig. 15 reference). In the opposite case, it can be considered that a barcode swapping phenomenon occurred, and the figure was smaller than or equal to the rate of the index hopping phenomenon that generally exists in the Illumina NGS platform. In other words, even after going through the bMDA-seq process, the barcode swapping phenomenon was measured to be negligible.

### Example 2. Confirmation of barcode bias phenomenon and suggestion of solution

### (1) Confirmation of barcode bias phenomenon

It was confirmed that three types of barcode bias phenomena appear when performing bMDA using a barcode-containing primer.

### 1) bMDA amplification bias

bMDA amplification bias refers to a phenomenon in which the MDA reaction occurs more actively around the relevant genomic region when the barcode sequence of the barcode-containing primer is complementary to the sequence of the target sample.

### 2) Bias in the amount of barcoding DNA contained in bMDA amplification products

This means that depending on the barcode sequence, the strength of the interaction between the barcode-containing primer and DNA polymerase or the probability of the primer hybridizing to the target sample during the bMDA reaction may vary, and accordingly, this means that the amount of barcoded DNA in the bMDA amplification product is different for each barcode.

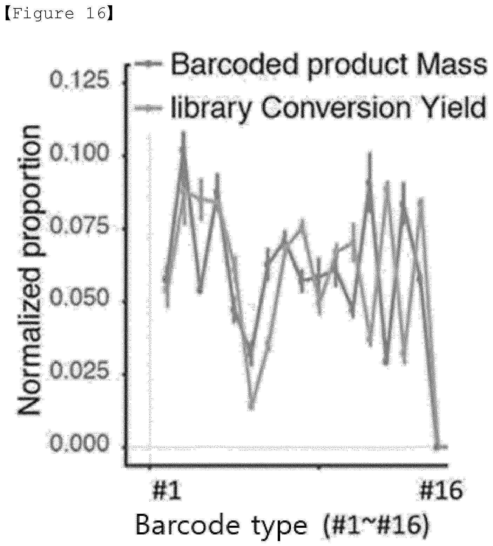

After performing bMDA by designing barcode-containing primers with 16 different barcode sequences, the ratio of the concentration of dsDNA immediately after bMDA and the concentration of dsDNA after biotin purification was measured(Barcoded product Mass). As a result, it was confirmed that there was a bias in the amount of barcoding DNA contained in the bMDA amplification product for each barcode (see Fig. 16). The sequences of the 16 barcode-containing primers are shown in Table 4 below.

(In Table 4, /5Biosg/ is a 5'-biotin modification and is a capture material, NNNNNN is a binding region composed of 6 random bases, and * is a phosphorothiolate modified nucleotide that is resistant to 3'-5' nucleic acid exonuclease reaction.)

**[Table 4]**

| Primer Sequence | |
|---|---|
| /5Biosg/TAGTCNNNN*N*N(SEQ ID NO: 56) | /5Biosg/AGAAGNNNN*N*N(SEQ ID NO: 57) |
| /5Biosg/ACCGANNNN*N*N(SEQ ID NO: 58) | /5Biosg/TGGCTNNNN*N*N(SEQ ID NO: 59) |
| /5Biosg/AGCTANNNN*N*N(SEQ ID NO: 60) | /5Biosg/GCTCTNNNN*N*N(SEQ ID NO: 61) |
| /5Biosg/GTGACNNNN*N*N(SEQ ID NO: 62) | /5Biosg/ACAGTNNNN*N*N(SEQ ID NO: 63) |
| /5Biosg/ATGCCNNNN*N*N(SEQ ID NO: 64) | /5Biosg/TACTGNNNN*N*N(SEQ ID NO: 65) |
| /5Biosg/TCAAGNNNN*N*N(SEQ ID NO: 66) | /5Biosg/CGTTANNNN*N*N(SEQ ID NO: 67) |
| /5Biosg/CCTGGNNNN*N*N(SEQ ID NO: 68) | /5Biosg/TTACCNNNN*N*N(SEQ ID NO: 69) |
| /5Biosg/GATGTNNNN*N*N(SEQ ID NO: 70) | /5Biosg/CTACANNNN*N*N(SEQ ID NO: 71) |

### 3) NGS library conversion efficiency bias

This means that the Library Conversion Yield is different for each barcode (Because the efficiency of end-repair, A-tailing, or ligation varies depending on the barcode sequence when the NGS library preparation process is performed after selectively capturing barcoded DNA) (see Fig. 16).

### (2) Suggestion of a solution to the barcode bias phenomenon

When samples are pooled without considering the barcode bias phenomenon, the amount of barcoded DNA present in the same volume varies depending on the barcode sequence, and when this is sequenced, the number of reads is not uniform for each sample. To solve this, the following methods can be considered.

### 1) Way to adjust the ratio of barcode-containing primer

The barcode bias phenomenon can be resolved by performing bMDA by adjusting the ratio of barcode-containing primers by considering the amount of barcoded DNA contained in the bMDA amplification product or the NGS library conversion efficiency in advance when mixing barcode-containing primer and random hexamer at a specific ratio.

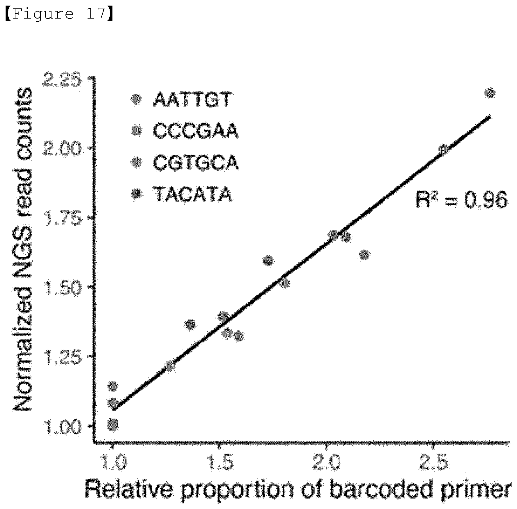

It was experimentally confirmed that as the ratio of barcode-containing primers in the bMDA reaction solution(Relative proportion of barcoded primer) increased, the number of NGS reads(Normalized NGS read counts) when NGS sequencing was performed after bMDA-seq library preparation relatively increased(see Fig. 17). Through this, it was verified that as the ratio of barcode-containing primers increases, the amount of barcoding DNA in the bMDA amplification product can be increased. Therefore, if the amplification products obtained by appropriately adjusting the ratio of each barcode-containing primer are mixed in the same volume, the amount of barcoded DNA is almost constant for each type of barcode-containing primer, or the number of NGS reads can be made almost constant for each barcode-containing primer type, solving the barcode bias phenomenon.

In order to verify the effectiveness of the above method, the degree of barcode bias was measured when the ratios of barcode-containing primers were all set constant and when each ratio was set differently. Specifically, bMDA was performed on 66 pg of HL-60 gDNA using 48 different barcode-containing primers. In the experimental group where the ratio of barcode-containing primers was constant, 2% barcode-containing primer was used, and in the experimental group where the ratio of barcode-containing primers was appropriately adjusted, the ratios shown in Table 5 below were used.

(In Table 5, /5Biosg/ is a 5'-biotin modification and is a capture material, NNNNNN is a binding region composed of 6 random bases, and * is a phosphorothiolate modified nucleotide that is resistant to 3'-5' nucleic acid exonuclease reaction.)

**[Table 5]**

| Primer Sequence | Ratio | Primer Sequence | Ratio |
|---|---|---|---|
| /5Biosg/ACTCTGNNNN*N*N(SEQ ID NO: 8) | 1.30% | /5Biosg/CTTCCGNNNN*N*N(SEQ ID NO: 9) | 1.76% |
| /5Biosg/CGAACTNNNN*N*N(SEQ ID NO: 10) | 1.18% | /5Biosg/CCCGAANNNN*N*N(SEQ ID NO: 11) | 1.32% |
| /5Biosg/CGGCTTNNNN*N*N(SEQ ID NO: 12) | 0.98% | /5Biosg/CACGGCNNNN*N*N(SEQ ID NO: 13) | 1.02% |
| /5Biosg/CTAGCCNNNN*N*N(SEQ ID NO: 14) | 1.30% | /5Biosg/CGTGCANNNN*N*N(SEQ ID NO: 15) | 0.92% |
| /5Biosg/CTATATNNNN*N*N(SEQ ID NO: 16) | 1.44% | /5Biosg/CCCTTGNNNN*N*N(SEQ ID NO: 17) | 1.62% |
| /5Biosg/CTTAGANNNN*N*N(SEQ ID NO: 18) | 0.94% | /5Biosg/CTCATTNNNN*N*N(SEQ ID NO: 19) | 1.54% |
| /5Biosg/AAACGANNNN*N*N(SEQ ID NO: 20 | 1.06% | /5Biosg/ATCACCNNNN*N*N(SEQ ID NO: 21) | 2.24% |
| /5Biosg/ATCCATNNNN*N*N(SEQ ID NO: 22) | 1.92% | /5Biosg/CCAAAGNNNN*N*N(SEQ ID NO: 23) | 0.82% |
| /5Biosg/ACATCGNNNN*N*N(SEQ ID NO: 24) | 1,840 | /5Biosg/ACTAACNNNN*N*N(SEQ ID NO: 25) | 1.68% |
| /5Biosg/CCTGTTNNNN*N*N(SEQ ID NO: 26) | 1.02% | /5Biosg/AGCTCANNNN*N*N(SEQ ID NO: 27) | 1.22% |
| /5Biosg/AATTGTNNNN*N*N(SEQ ID NO: 28) | 0.92% | /5Biosg/ACCAGANNNN*N*N(SEQ ID NO: 29) | 0.98% |
| /5Biosg/GTAGTTNNNN*N*N(SEQ ID NO: 30) | 1.48% | /5Biosg/TGAAACNNNN*N*N(SEQ ID NO: 31) | 1.02% |
| /5Biosg/TTATTGNNNN*N*N(SEQ ID NO: 32) | 1.12% | /5Biosg/AAAGCTNNNN*N*N(SEQ ID NO: 33) | 1.00% |
| /5Biosg/TATAAGNNNN*N*N(SEQ ID NO: 34) | 0.88% | /5Biosg/TTACGTNNNN*N*N(SEQ ID NO: 35) | 1.32% |
| /5Biosg/AATACANNNN*N*N(SEQ ID NO: 36) | 1.26% | /5Biosg/ATTTAGNNNN*N*N(SEQ ID NO: 37) | 1.60% |
| /5Biosg/TTCTACNNNN*N*N(SEQ ID NO: 38) | 2.44% | /5Biosg/AAGTAANNNN*N*N(SEQ ID NO: 39) | 0.90% |
| /5Biosg/TGACTANNNN*N*N(SEQ ID NO: 40) | 1.34% | /5Biosg/TTAACANNNN*N*N(SEQ ID NO: 41) | 1.46% |
| /5Biosg/TTGAATNNNN*N*N(SEQ ID NO: 42) | 0.72% | /5Biosg/ACGGTANNNN*N*N(SEQ ID NO: 43) | 1.32% |
| /5Biosg/TTTGCTNNNN*N*N(SEQ ID NO: 44) | 1.04% | /5Biosg/TTTCAANNNN*N*N(SEQ ID NO: 45) | 2.10% |
| /5Biosg/TACATANNNN*N*N(SEQ ID NO: 46) | 1.06% | /5Biosg/TCTAGTNNNN*N*N(SEQ ID NO: 47) | 1.62% |
| /5Biosg/TTGTGANNNN*N*N(SEQ ID NO: 48) | 0.78% | /5Biosg/TCATGCNNNN*N*N(SEQ ID NO: 49) | 1.18% |
| /5Biosg/TAAGTCNNNN*N*N(SEQ ID NO: 50) | 0.86% | /5Biosg/TAACCGNNNN*N*N(SEQ ID NO: 51) | 1.60% |
| /5Biosg/TCAGATNNNN*N*N(SEQ ID NO: 52) | 1.00% | /5Biosg/TCCCAGNNNN*N*N(SEQ ID NO: 53) | 1.48% |
| /5Biosg/TCGACGNNNN*N*N(SEQ ID NO: 54) | 1.64% | /5Biosg/TAGAGCNNNN*N*N(SEQ ID NO: 55) | 0.54% |

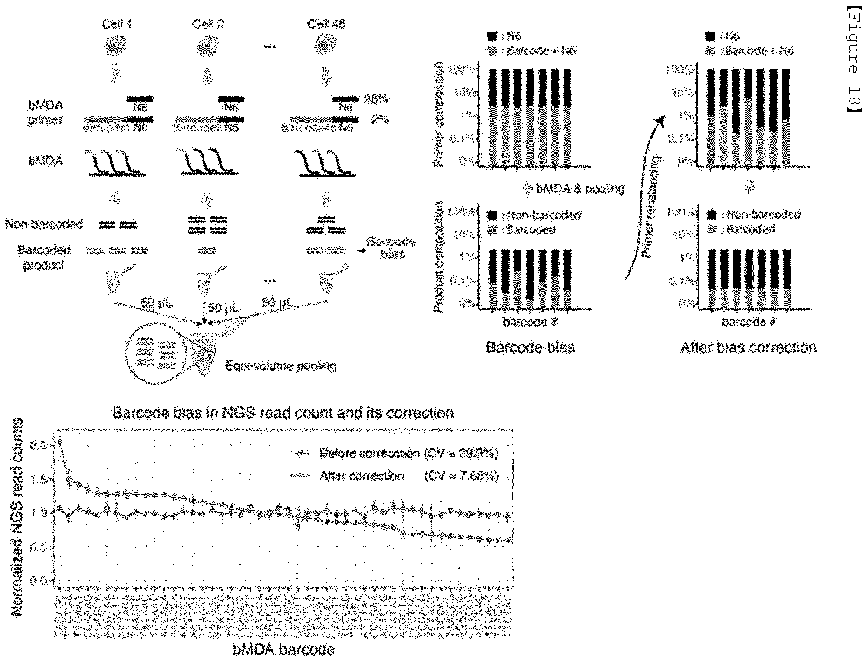

As a result, when the ratio of barcode-containing primers was all constant (2%), the coefficient of variation (CV) value of the number of NGS reads was about 29.9% (n=3) (Before correction), while when the ratio of barcode-containing primers was set differently to a specific ratio (After correction), a CV value of approximately 7.68% (n=3) was found (see Fig. 18). In the case of the Illumina library pooling method, which uniformly adjusts the number of NGS reads between different libraries based on the concentration and length of libraries prepared with different indexes in a typical NGS application, a CV value of about 8.53% is shown. Compared to this, the barcode bias phenomenon could be effectively eliminated by adjusting the barcode-containing primer ratio.

In addition, if you prepare a reagent in which the barcode-containing primer ratio is adjusted in this way, the number of NGS sequencing reads will be uniform even if NGS sequencing is performed after pooling all bMDA amplification products using different barcodes in the same volume. Therefore, user convenience can be maximized. In other words, users can conduct an uniform experiment process without considering differences according to barcode sequence.

In order to effectively control the ratio of barcode-containing primers, the amount of barcoding DNA contained in the bMDA amplification product or the NGS library conversion efficiency can be obtained in advance as data through experiment, or the value estimated through an algorithm for predicting this can be used as a standard. In addition, the ratio of primers can be adjusted so that the amount of barcoding DNA contained in the bMDA amplification product is almost constant for each sample. Considering both the amount of barcoding DNA contained in the bMDA amplification product and the NGS library conversion efficiency, the primer ratio can be adjusted so that the number of NGS reads is approximately constant for each sample.

In addition, an experiment is performed to determine whether there is a difference in the number of NGS reads when the amount of template used as input for bMDA increases or decreases in the case that barcode bias is corrected by adjusting the ratio of barcode-containing primers. Specifically, 5 to 16 primers were selected among the 48 different barcode-containing primers in Table 3 above, bMDA was performed using different amounts of HL-60 gDNA as a template, and then the coefficient of variation (CV) value of the number of NGS reads between barcodes within one group of pooled bMDA-seq products was measured.

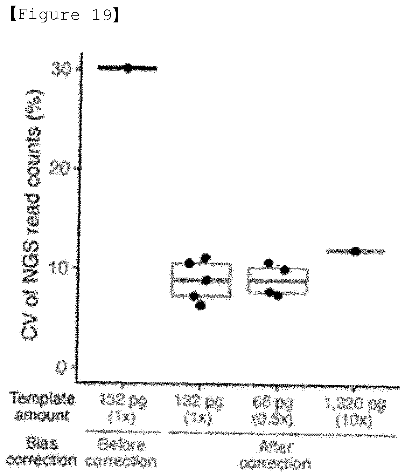

As a result of the experiment, it was confirmed that when the barcode expression was corrected by adjusting the ratio of the barcode-containing primer, there was no significant difference in the number of NGS reads even if the amount of input template increased or decreased (see Fig. 19). From the user's perspective, it is much more common to want to secure a similar level of NGS reads for each sample, so it can be a great advantage to be able to secure a similar number of NGS reads for each sample by mixing the amplification products in the same volume.

Through the above experiments, it was confirmed that if the ratio of barcode-containing primers is appropriately adjusted according to specific standards, the CV can be greatly reduced by solving the barcode bias phenomenon, and a similar number of NGS reads can be secured for each sample(or by single-cell) by mixing the amplification products in equal volumes.

### 2) Other solutions to barcode bias phenomenon

As a way to reduce bMDA bias according to barcode sequence, barcode sequences that do not exist in large quantities in the sample can be selected, or barcode sequences that do not significantly affect bMDA bias can be selected. Additionally, bMDA bias can be minimized by preparing only the barcode sequence region within the barcode-containing primer in dsDNA form.

Another method is to obtain the amount of barcoding DNA contained in the bMDA amplification product according to the barcode sequence or the NGS library conversion efficiency as data in advance through experiments, or to pool samples into different volumes to have the desired ratio for each sample through an algorithm that predicts this. At this time, the amount of barcoding DNA contained in the bMDA amplification product can be adjusted so that it is almost constant for each sample and pooled, and considering the amount of barcoding DNA contained in the bMDA amplification product and the NGS library conversion efficiency, the number of NGS reads may be adjusted and pooled for each sample to be approximately constant.

### Example 3. Confirmation of bMDA quality improvement effect of laser-based cell separation method

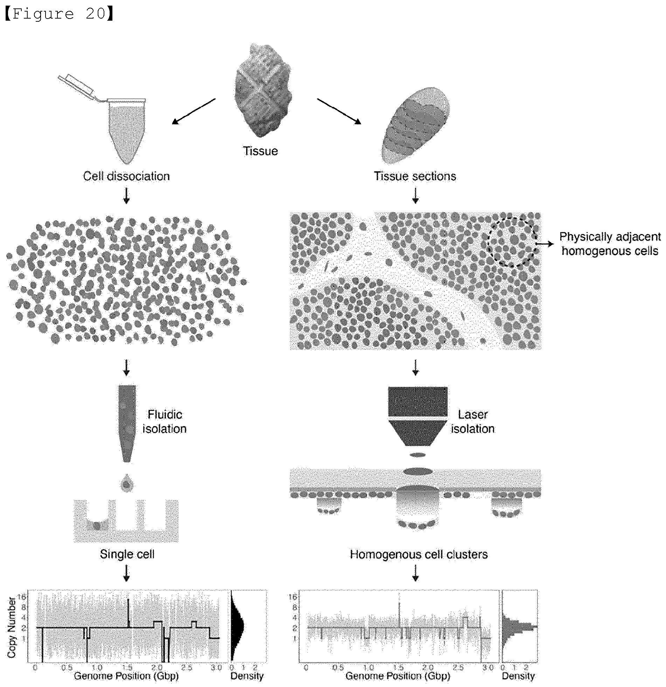

As a conventional single-cell isolation method, fluidic isolation was mainly used, but this method had the problem of poor amplification quality due to MDA amplification bias(see Fig. 20). As mentioned earlier, the reason is that due to the limitations of MDA amplification technology, when MDA is performed using a single-cell as a template, there are only 5 to 10% of cells with high amplification uniformity at a level that allows CNA analysis, and most cells show very serious amplification bias (see Fig. 11f).

To solve the above problems, a method of performing bMDA at once by separating multiple cells with similar genome information rather than a single cell using a laser can be used. Cell separation using a laser is accomplished by observing the above-mentioned ITO-coated slide glass under a microscope and then irradiating an infrared laser to the area where the cells to be separated exist. Taking cancer tissue as an example, spatially adjacent cancer cells have relatively similar (homogeneous) genome information compared to spatially distant cells. Therefore, by performing bMDA on multiple cells by separating physically adjacent cells rather than single-cell, the problem of bMDA amplification bias can be innovatively improved (see Fig. 20).

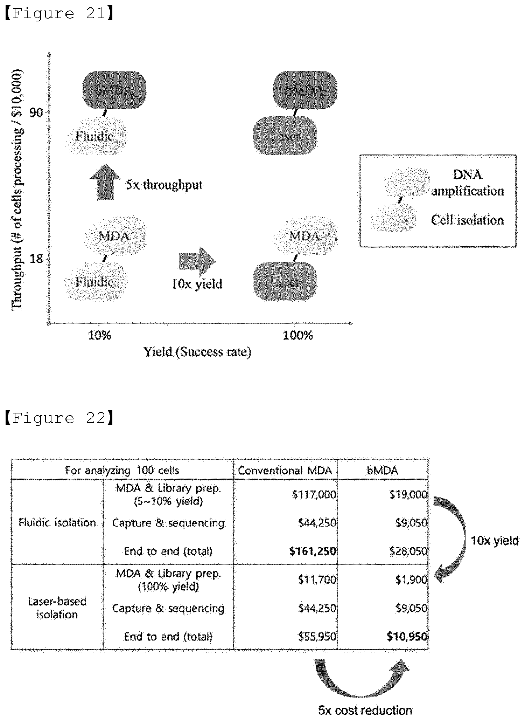

In this way, when using a method of separating the region of interest using a laser, the success rate (yield) can be increased to 100% compared to the method using fluid, which is the existing single-cell separation method (see Fig. 21). The reason is that the bMDA amplification bias can be lowered to a level where CNA analysis is possible by looking at only about 10 genetically similar cells (see Fig. 11f). In addition, because bMDA has about 5 times higher throughput than MDA (see Fig. 21). It was confirmed that when using bMDA and laser-based cell separation methods, a total cost reduction effect of about 15 times was observed compared to cell separation methods using MDA and fluid (see Fig. 22).

Through the above results, it was confirmed that the laser-based cell separation method can minimize the amplification bias problem of bMDA and provide high-quality amplification products by separating and analyzing physically adjacent cells together. This is because physically adjacent cells have higher genetic similarity than physically distant cells, and as the amount of input gDNA used for bMDA increases, amplification bias decreases and amplification quality increases. In other words, the laser-based cell separation method does not separate single-cells, but can exert a similar effect to analyzing single-cells while separating multiple cells by utilizing similarities between physically adjacent cells. Through this, the proportion of amplification products that do not pass quality control (QC), which was a problem in single-cell MDA, can be reduced from 90 to 95% to 0%, and the cost inefficiency problem of single-cell MDA can be reduced.

In addition, the laser-based cell separation method is not only used as an approach to separate physically adjacent cells, but also an approach that classifies cells based on various information such as size, shape, color, and fluorescent signal that can be obtained through observation of microscope images, and then separates cells included in the classification to be analyzed at once. For example, by observing a blood smear slide under a microscope, a specific type of white blood cell to be isolated can be separated, or by observing FISH (fluorescence in situ hybridization)-stained slides with a fluorescence microscope, cells showing signals to be isolated can be separated and collected, and then bMDA can be performed on several cells at once.

In conclusion, the improvement in amplification quality of the laser-based cell separation method works in synergy with the throughput improvement and price reduction of bMDA. Therefore, it was proven to be a very efficient method that solves the problems of low quality and low parallelism of single-cell genome analysis for users and reduces the amplification bias problem.

## Claims

1. A nucleic acid amplification method, comprising the step of amplifying nucleic acids by adding a first primer, a second primer and a nucleic acid polymerase to a sample containing a target nucleic acid, wherein
the first primer is a polynucleotide consisting of a base sequence including a first base sequence that hybridizes to the target nucleic acid; and a fixed second base sequence that distinguishes the sample from which the target nucleic acid is derived, and
the second primer is a polynucleotide consisting of a base sequence including a third base sequence that hybridizes to the target nucleic acid.

2. The nucleic acid amplification method according to claim 1, wherein the number of bases in the first base sequence or the third base sequence is an integer each independently selected from 4 to 10.

3. The nucleic acid amplification method according to claim 1, wherein the bases constituting the first base sequence or the third base sequence are bases selected from the group consisting of A (adenine), T (thymine), G (guanine), C (cytosine), and modifications thereof.

4. The nucleic acid amplification method according to claim 1, wherein the number of bases in the second base sequence is an integer selected from 4 to 35.

5. The nucleic acid amplification method according to claim 1, wherein the first primer is added at 0.1 to 10 mol% compared to the second primer.

6. The nucleic acid amplification method according to claim 1, wherein the first primer contains a substance that inhibits the affinity between the nucleic acid polymerase and the first primer.

7. The nucleic acid amplification method according to claim 1, wherein the first primer or the second primer contains a base that is resistant to nuclease.

8. The nucleic acid amplification method according to claim 1, which further comprises the step of selecting the nucleic acid amplified with the first primer.

9. The nucleic acid amplification method according to claim 8, wherein the first primer is combined with a capture material.

10. The nucleic acid amplification method according to claim 9, wherein the capture material is at least one selected from the group consisting of biotin, D-biotin, biotin dT, biotin-TEG, dual biotin, PC-biotin, acrylic, thiol, dithiol, amine group, acrylic group, NHS ester, azide, hexynyl, octadiynyl dU, his tag, protein tag, polyA (poly adenine), at least 10 base sequences among the base sequences that make up the NGS sequencing adapter, and a polynucleotide consisting of at least 10 base sequences.

11. The nucleic acid amplification method according to claim 8, wherein the selection is performed by a method using at least one substance selected from the group consisting of avidin, neutravidin, streptavidin, carboxylic group, thiol, alkyne, digoxigenin, azide and hexynyl, or a polymerase chain reaction (PCR) method.

12. The nucleic acid amplification method according to claim 8, wherein the selection involves selecting double-stranded or single-stranded nucleic acids.

13. The nucleic acid amplification method according to claim 1, which further comprises the steps of:
purification of the nucleic acid amplified from the sample;
fragmentation of the purified nucleic acid; and
preparation of Next Generation Sequencing (NGS) library using the fragmented nucleic acid.

14. The nucleic acid amplification method according to claim 1, wherein the sample is separated by at least one technique selected from the group consisting of spatially resolved technique, dielectrophoretic digital sorting, fluorescence activated cell sorting(FACS), hydrodynamic traps, droplet, microwell, microfluidics, micromanipulation, Raman tweezers and CellRaft.

15. The nucleic acid amplification method according to claim 14, wherein the spatially resolved technique is at least one technique selected from the group consisting of Laser capture microdissection (LCM), laser microdisection (LMD), Laser pressure catapulting (LPC) and phenotype-based high-throughput laser-aided isolation and sequencing (PHLI-seq).

16. The nucleic acid amplification method according to claim 1, wherein the nucleic acid polymerase is DNA polymerase or RNA polymerase.

17. The nucleic acid amplification method according to claim 16, wherein the DNA polymerase is at least one DNA polymerase selected from the group consisting of phi29 DNA polymerase, Tts DNA polymerase, phase M2 DNA polymerase, VENT DNA polymerase, Klenow fragment of DNA polymerase I, T5 DNA polymerase, PRD1 DNA polymerase, T4 DNA polymerase holoenzyme, T7 polymerase derived T7 Sequenase and Bst DNA polymerase.

18. The nucleic acid amplification method according to claim 1, wherein
the samples are plural and different from each other,
the target nucleic acids are plural and different from each other,
the first primers are plural and different from each other,
the second primers are plural and different from each other, and
the first primers, which are plural and different from each other, are polynucleotides composed of base sequences including the second base sequences, which are fixed and different from each other.

19. The nucleic acid amplification method according to claim 18, wherein the first primer is added at 0.1 to 10 mol% compared to the second primer.

20. The nucleic acid amplification method according to claim 18, which further comprises the step of selecting the nucleic acids amplified with the plurality of the first primers.

21. The nucleic acid amplification method according to claim 20, wherein the plurality of the first primers are combined with a capture material.

22. The nucleic acid amplification method according to claim 18, which further comprises the steps of:
purification of the nucleic acids amplified from the plurality of the samples;
fragmentation of the purified nucleic acids;
pooling of the fragmented nucleic acids; and
preparation of Next Generation Sequencing (NGS) library using the pooled nucleic acids.

23. A primer set for nucleic acid amplification comprising a first primer and a second primer, wherein
the first primer is a polynucleotide consisting of a base sequence including a first base sequence that hybridizes to the target nucleic acid; and a fixed second base sequence that distinguishes the sample from which the target nucleic acid is derived, and
the second primer is a polynucleotide consisting of a base sequence including a third base sequence that hybridizes to the target nucleic acid.

24. The primer set according to claim 23, wherein
the first primers are plural and different from each other,
the second primers are plural and different from each other, and
the first primers, which are plural and different from each other, are polynucleotides composed of base sequences including the second base sequences, which are fixed and different from each other.

25. The primer set according to claim 24, wherein the first primer in the primer set is added at 0.1 to 10 mol% compared to the second primer.

26. A kit for nucleic acid amplification comprising a first primer and a second primer, wherein
the first primer is a polynucleotide consisting of a base sequence including a first base sequence that hybridizes to the target nucleic acid; and a fixed second base sequence that distinguishes the sample from which the target nucleic acid is derived, and
the second primer is a polynucleotide consisting of a base sequence including a third base sequence that hybridizes to the target nucleic acid.

27. The kit according to claim 26, wherein
the first primers are plural and different from each other,
the second primers are plural and different from each other, and
the first primers, which are plural and different from each other, are polynucleotides composed of base sequences including the second base sequences, which are fixed and different from each other.

28. The kit according to claim 27, wherein the first primer in the kit is added at 0.1 to 10 mol% compared to the second primer.

29. The kit according to claim 26, wherein the kit further comprises a well, and
the first primer and the second primer are seeded into the well.

30. The kit according to claim 29, wherein the well is at least one selected from the group consisting of a 8-strip tube, a 96-well plate and a 384 plate.

31. The kit according to claim 26, wherein the first primer and the second primer are freeze-dried.

32. The kit according to claim 26, wherein the first primer and the second primer are combined into beads with a diameter of 1 um to 10 mm.
